# EUROPEAN PATENT APPLICATION

(11) **EP 4 656 189 A1**
(43) Date of publication of application: **03.12.2025**
(21) Application number: 24749718.3
(22) Date of filing: 31.01.2024
(51) Int. Cl.: A61K 31/138, A61K 9/00, A61K 9/06, A61K 9/08, A61K 9/10, A61P 17/06

(54) **COMPOUND AND USE THEREOF**

(30) Priority: 01.02.2023 CN 202310049428
(71) Applicant: Benethera (Shaoxing) Biotechnology Co., Ltd., Shaoxing, Zhejiang 312025 (CN)
(72) Inventor: GENG, Shuang, Beijing 100085 (CN); KANG, Ning, Beijing 100085 (CN)
(74) Representative: Bayramoglu et al.
(86) International application number: PCT/CN2024/075027
(87) International publication number: WO 2024/160239

(57) **Abstract**

The present invention provides a compound represented by formula I or a pharmaceutically acceptable salt, stereoisomer, isotope derivative, solvate (such as hydrate), prodrug, metal chelate, crystal form or metabolite thereof, and a use thereof in the field of medicines. The compound, especially propafenone, can be used for treating and/or preventing psoriasis diseases. According to the present invention, it is further found that under the condition of a same administration dosage, in treating psoriasis diseases, the effect of administration by application is better than the effect of administration by injection. Compared with flecainide ointments having similar anti-arrhythmia functions, a propafenone ointment of the present invention has a better effect on treating psoriasis diseases.

## Description

### TECHNICAL FIELD

The present invention relates to the technical field of biological medicine, specifically to use of a compound represented by formula I or a pharmaceutically acceptable salt, stereoisomer, isotope derivative, solvate (such as hydrate), prodrug, metal chelate, crystal form, or metabolite thereof in treating and/or preventing psoriasis diseases.

### BACKGROUND TECHNOLOGY

Psoriasis is caused by the body's immune system abnormalities caused by the skin symptoms. At present, the main clinical treatment methods are ultraviolet irradiation, oral immunosuppressants, and topical immunosuppressants.

Patent CA2950727A1 discloses the preparation of β-d-nicotinamide nucleosides in the nicotinic acid pathway, which can be used in the preparation of a variety of drugs, and in the treatment of various diseases including psoriasis. Patent CA2618360C discloses benzothiazoles and thiazolopyridines as sirtuin modulators which can be used in the preparation of a variety of drugs and in the treatment of a variety of diseases including psoriasis.

However, the prior art does not report on the role of the compound of Formula I in the treatment and/or prevention of psoriasis-like diseases.

### CONTENT OF THE INVENTION

The present invention provides a compound of formula I or a pharmaceutically acceptable salt, stereoisomer, hydrate, prodrug, solvate, metabolite, or isotope derivative thereof, and use thereof in the treatment or prevention of psoriatic diseases.

In a first aspect of the present invention, provided is the use of a compound represented by formula I or a pharmaceutically acceptable salt, stereoisomer, isotope derivative, solvate (such as hydrate), prodrug, metal chelate, crystal form or metabolite thereof in the preparation of a drug for treating and/or preventing psoriasis-like disease, the compound having the following structure: 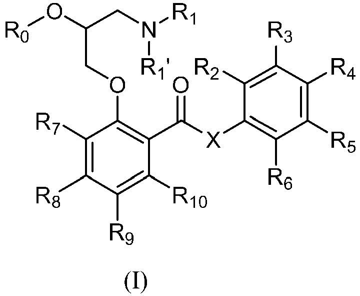
wherein X is selected from a single bond, -O-(C₀-C₁₀ alkylene)-, -S-(C₀-C₁₀ alkylene)-, -N(C₀-C₁₀ alkyl)-(C₀-C₁₀ alkylene)-, C₁-C₁₀ alkylene, C₂-C₁₀ alkenylene, C₂-C₁₀ alkynylene;
R₀ is selected from H, -CO(C₁₋₁₀ alkyl), -CO(C₆₋₁₀ aryl);
R₁ and R₁' are independently selected from H, C₁-C₁₀ alkyl, C₂-C₁₀ alkenyl, C₂-C₁₀ alkynyl, C₁-C₁₀ haloalkyl, -(C₀-C₆ alkylene)-(C₃-C₁₀ cycloalkyl), -(C₀-C₆ alkylene)-(C₆-C₁₀ aryl), -(C₀-C₆ alkylene)-(4-10 membered heterocyclyl), -N(C₀₋₁₀ alkyl) (C₀₋₁₀ alkyl), -O(C₀₋₁₀ alkyl); or R₁ and R₁' together with the nitrogen atom to which they are attached form a heterocyclic ring, wherein H on the heterocyclic ring is optionally substituted by one or more groups selected from halogen, cyano, nitro, azido, C₁-C₁₀ alkyl, C₁-C₁₀ haloalkyl, -N(C₀₋₁₀ alkyl) (C₀₋₁₀ alkyl), -O(C₀₋₁₀ alkyl), -S(C₀₋₁₀ alkyl);
R₂, R₃, R₄, R₅, R₆, R₇, R₈, R₉, and R₁₀ are independently selected from H, halogen, cyano, nitro, azido, C₁-C₁₀ alkyl, C₁-C₁₀ haloalkyl, -(C₀-C₆ alkylene)-(C₃-C₁₀ cycloalkyl), -(C₀-C₆ alkylene)-(C₆-C₁₀ aryl), -(C₀-C₆ alkylene)-(4-10 membered heterocyclyl), -N(C₀₋₁₀ alkyl) (C₀₋₁₀ alkyl), -O (C₀₋₁₀ alkyl), -S (C₀₋₁₀ alkyl).
Further, X is selected from C₁-C₆ alkylene (e.g. -CH₂-, -CH₂CH₂-, -CH₂CH₂CH₂-, -CH₂CH₂CH₂CH₂-, -CH₂CH₂CH₂CH₂CH₂-, -CH₂CH₂CH₂CH₂CH₂CH₂-), C₂-C₆ alkenylene (-CH=CH-, -CH=CH-CH=CH-, -CH=CH-CH=CH-CH=CH-).

In some embodiments of the present invention, X is -CH₂CH₂- or -CH=CH-.

Further, R₀ is selected from H, -CO(C₁₋₆ alkyl) (e.g. -COCH₃, -COCH₂CH₃, -COCH₂CH₂CH₃, -COCH₂CH₂CH₂CH₃).

In some embodiments of the present invention, R₀ is H.

In some embodiments of the present invention, R₀ is -COCH₃.

Further, R₁ and R₁' are independently selected from H, C₁-C₆ alkyl (e.g. -CH₃, -CH₂CH₃, -CH₂CH₂CH₃, -CH(CH₃)₂), -(C₀-C₆ alkylene)(C₆-C₁₀ aryl) (e.g.

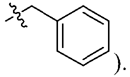

In some embodiments of the present invention, R₁' is H.

In some embodiments of the present invention, R₁ is -CH₂CH₂CH₃, and R₁' is H.

In some embodiments of the present invention, R₁ is -CH(CH₃)₂, and R₁' is H.

In some embodiments of the present invention, R₁ is -CH₂CH₃, and R₁' is -CH₃.

In some embodiments of the present invention, R₁ is 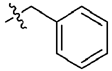 and R₁' is H; or, R₁ is H and R₁' is

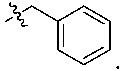

Further, R₁ and R₁' together with the nitrogen atom to which they are attached form a 4-6 membered nitrogen-containing heterocyclic ring, wherein H on the 4-6 membered nitrogen-containing heterocyclic ring is optionally substituted with one or more groups selected from C₁-C₆ alkyl.

Further, the 4-6 membered nitrogen-containing heterocycle is selected from: 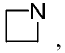 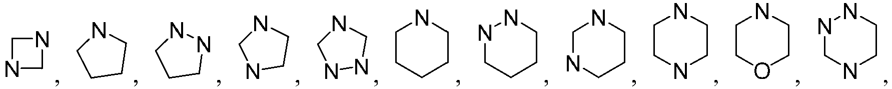 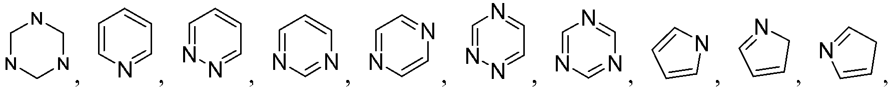 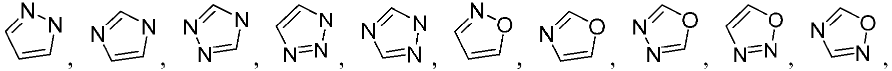 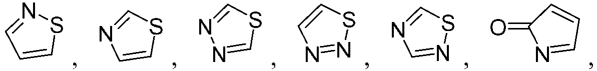 preferably,

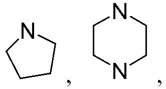

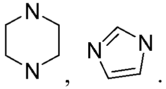

In some embodiments of the present invention, R₁ and R₁', together with the nitrogen atom to which they are attached, form a 4-6 membered nitrogen-containing heterocyclic ring,

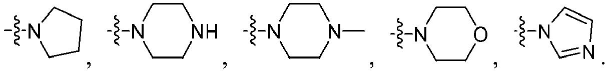

Further, R₂, R₃, R₄, R₅, R₆, R₇, R₈, R₉, and R₁₀ are independently selected from H, halogen, C₁-C₆ alkyl, C₁-C₆ haloalkyl, -O(C₀₋₆ alkyl).

In some embodiments of the present invention, R₂ is H.

In some embodiments of the present invention, R₃ is H.

In some embodiments of the present invention, R₄ is H, halogen (such as F, Cl, Br, I), or C₁-C₆ haloalkyl (such as -CF₃).

In some embodiments of the present invention, R₅ is H.

In some embodiments of the present invention, R₆ is H.

In some embodiments of the present invention, R₇ is H or -OH.

In some embodiments of the present invention, R₈ is H.

In some embodiments of the present invention, R₉ is H or -OH.

In some embodiments of the present invention, R₁₀ is H.

In some embodiments of the present invention, the compound has the following structure:

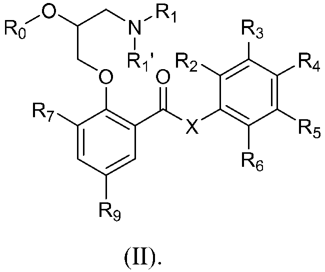

In some embodiments of the present invention, the compound has the following structure:

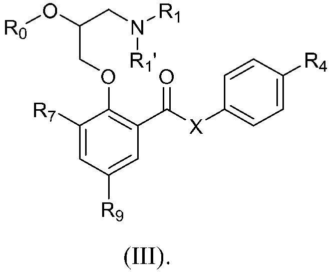

In some embodiments of the present invention, the compound has the following structure: 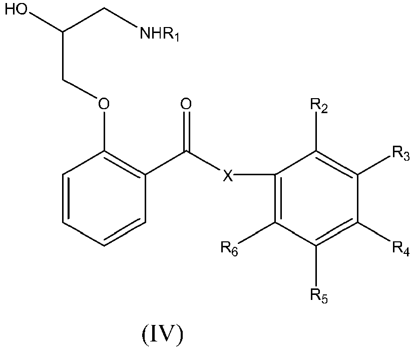 wherein X is selected from a single bond, -O-, -S-, or alkylene.

Preferably, in Formula IV, X is selected from a single bond or C₁-C₁₀ alkylene. Further preferably, X is selected from C₁-C₄ alkylene.

In Formula IV, R₁ is selected from H, alkyl, alkoxy, alkenyl, or alkynyl. Preferably, R₁ is selected from a C₁-C₁₀ alkyl. Further preferably, R₁ is selected from a C₁-C₄ alkyl.

In Formula IV, R₂, R₃, R₄, R₅, and R₆ are independently selected from H, alkyl, or halogen atoms. Preferably, R₂, R₃, R₄, R₅, and R₆ are independently selected from H, F, Cl, Br, I, methyl, or ethyl. Further preferably, R₂, R₃, R₄, R₅, and R₆ are independently selected from H, F, Cl, Br, I, methyl, or ethyl.

In some embodiments of the present invention, in Formula IV, X is selected from C₁-C₄ alkylene; R₁ is selected from C₁-C₄ alkyl; R₂, R₃, R₄, R₅, and R₆ are independently selected from H, F, Cl, Br, I, methyl, or ethyl.

In some embodiments of the present invention, in Formula IV, X is -CH₂CH₂-, and R₁ is selected from C₁-C₄ alkyl; R₂, R₃, R₄, R₅, and R₆ are independently selected from H, F, Cl, Br, I, methyl, or ethyl.

In some embodiments of the present invention, in Formula IV, X is selected from C₁-C₄ alkylene; R₁ is selected from -CH₂CH₂CH₃; R₂, R₃, R₄, R₅, and R₆ are independently selected from H, F, Cl, Br, I, methyl, or ethyl.

In some embodiments of the present invention, in Formula IV, X is selected from C₁-C₄ alkylene; R₁ is selected from C₁-C₄ alkyl; R₂, R₃, R₄, R₅, and R₆ are all H.

In some embodiments of the present invention, the compound has the following structure:

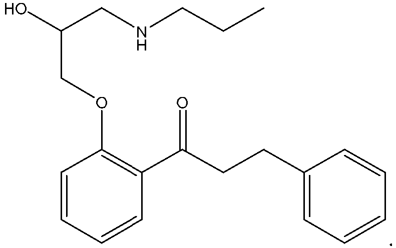

In other still embodiments of the present invention, the compound has the following structures:

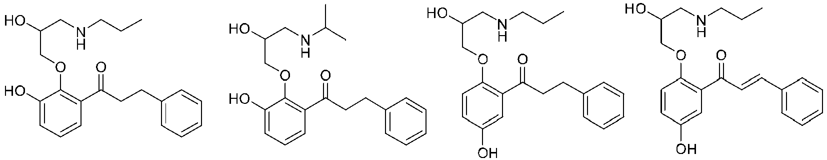

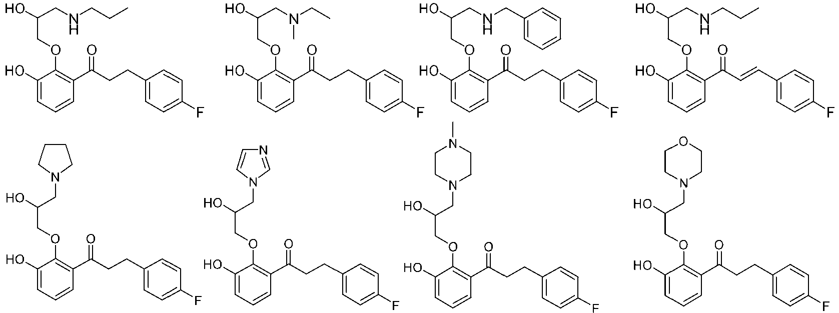

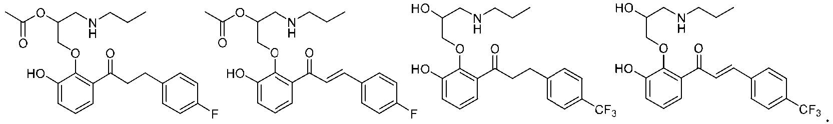

Further, the stereoisomer has the following structures:

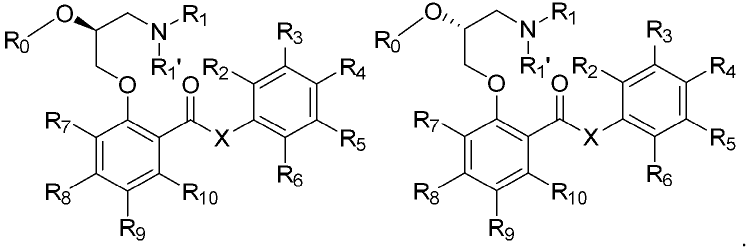

In still other embodiments of the present invention, the stereoisomer has the following structures:

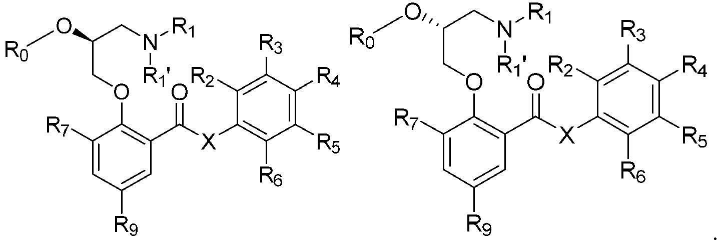

In still other embodiments of the present invention, the stereoisomer has the following structures:

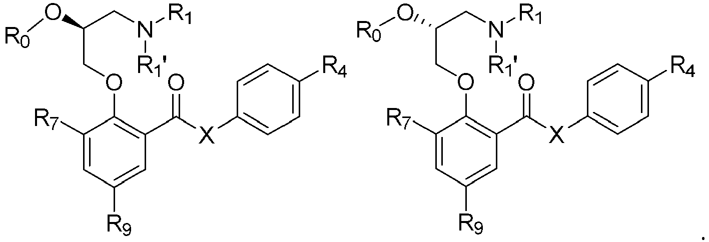

In a specific embodiment of the present invention, the stereoisomer of the compound has the following structures: 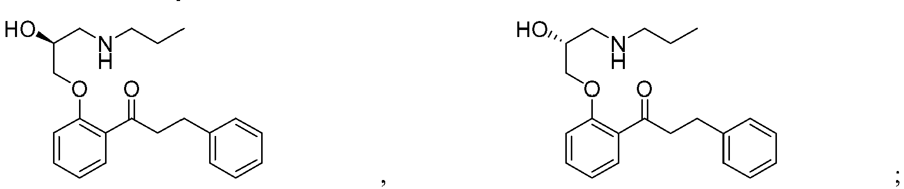 particularly

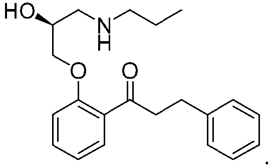

In other embodiments of the present invention, the stereoisomer of the compound has the following structures:

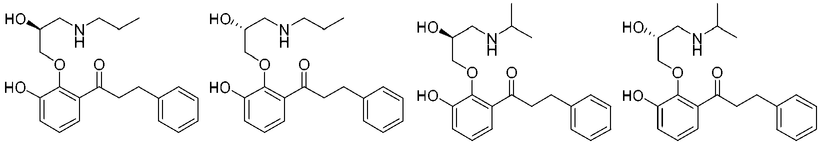

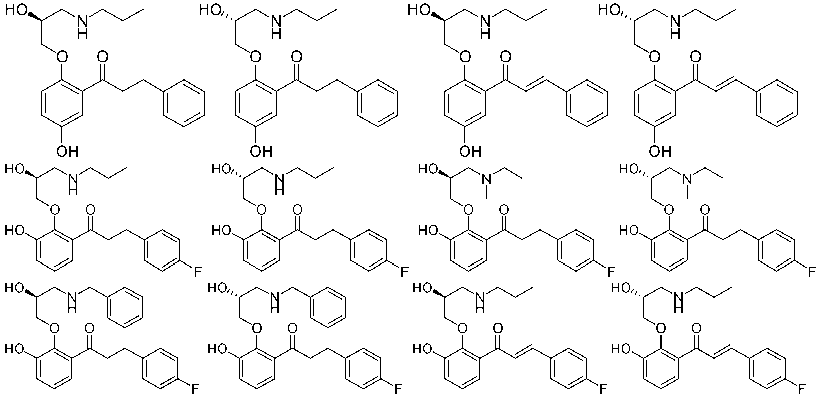

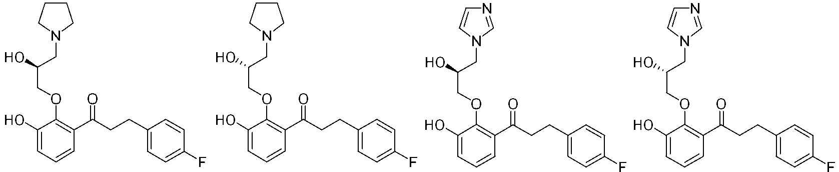

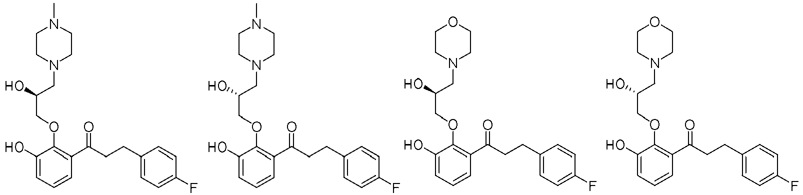

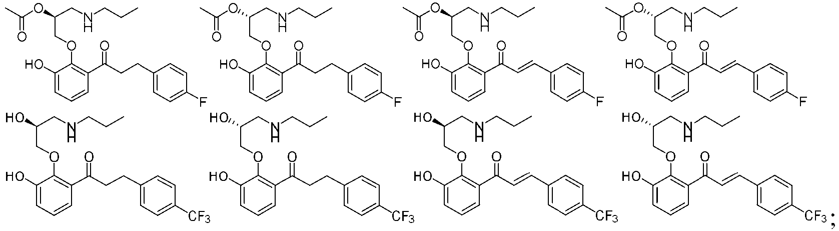

preferably, the stereoisomer has the following structures:

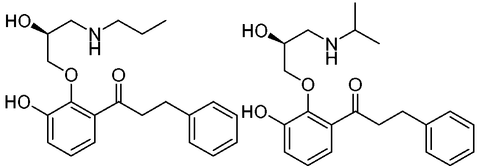

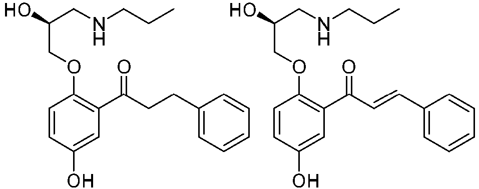

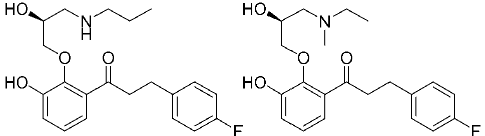

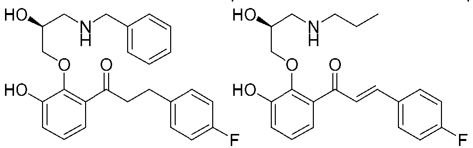

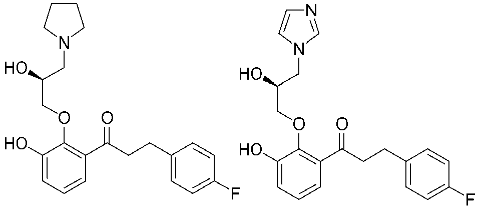

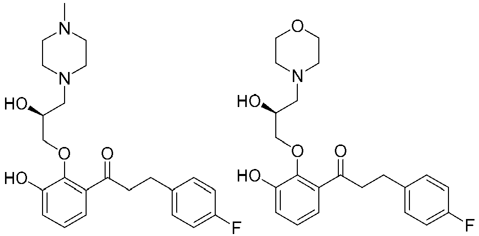

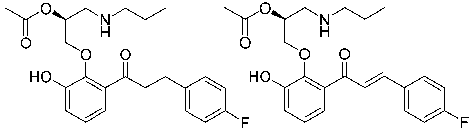

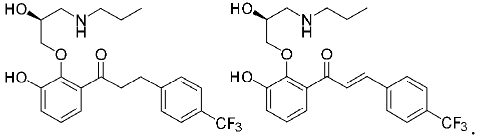

Further, the isotope derivative includes compounds wherein at least one atom is replaced by an atom having the same atomic number but a different atomic mass, such as stable and radioactive isotopes of hydrogen, carbon, oxygen, and the like, specifically 2H (deuterium, D), 3H (tritium, T), 11C, 13C, 14C, 170, 180, and the like, preferably deuterium.

In some embodiments of the present invention, the isotope derivative has the following structures:

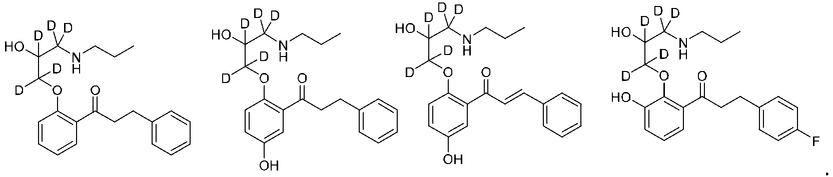

Further, the product is a drug including the compound represented by formula I or a pharmaceutically acceptable salt, stereoisomer, isotope derivative, solvate (such as hydrate), prodrug, metal chelate, crystal form or metabolite thereof;

The drug may include 0.01-99.5% by weight (in particular, 0.01%, 0.02%, 0.025%, 0.03%, 0.04%, 0.05%, 0.06%, 0.07%, 0.08%, 0.09%, 0.1%, 0.12%, 0.14%, 0.16%, 0.18%, 0.2%, 0.22%, 0.24%, 0.26%, 0.28%, 0.3%, 0.32%, 0.34%, 0.36%, 0.38%, 0.4%, 0.42%, 0.44%, 0.46%, 0.48%, 0.5%, 0.52%, 0.54%, 0.56%, 0.58%, 0.6%, 0.62%, 0.64%, 0.66%, 0.68%, 0.7%, 0.75%, 0.8%, 0.85%, 0.9%, 0.95%, 1%, 2%, 3%, 4%, 5%, 6%, 7%, 8%, 9%, 10%, 20%, 30%, 40%, 50%, 60%, 70%, 80%, 90%, 95%, 99%, 99.5%), preferably 0.02-0.5%, more preferably from 0.1%-0.5%, of the compound of formula I or a pharmaceutically acceptable salt, stereoisomer, isotopic derivative, solvate (e.g. hydrate), prodrug, metal chelate, crystalline form or metabolite thereof.

Further, the drug may be administered via any suitable administration route, such as gastrointestinal administration (e.g. oral) or parenteral administration (e.g. intravenous, intramuscular, subcutaneous, intradermal, intraorgan, intranasal, intraocular, instillation, intracerebral, intrathecal, transdermal, intrarectal, etc.), preferably parenteral administration.

In some embodiments of the present invention, the route of administration is subcutaneous, intradermal, or transdermal, in particular transdermal.

Furthermore, the drug may be in any suitable dosage form, such as a gastrointestinal administration dosage form or a parenteral administration dosage form, preferably a parenteral administration dosage form.

Further, the drug is in the dosage form of a topical preparation, an injection, or an oral preparation;
the topical preparation is selected from an aqueous solution, tincture, spirit, paste, oil, ointment, oil-in-water cream, oil ointment, water-in-oil cream, gel, film forming agent, paint, liniment, lotions, spray, suspension, solution, emulsion, microemulsion, suspension, plaster, gel, patch, powder aerosol, suppository, eye drops, or nasal drops;
the injection is selected from an aqueous, solution, suspension, emulsion, or powder injection;
the oral preparation is selected from a tablet, pill, dropping pill, powder, granule, capsule, troche, syrup, suspension, emulsion, microemulsion, powder aerosol, paste, sublingual tablet, or suppository.

In some embodiments of the present invention, the dosage form of the drug is a topical preparation.

In some embodiments of the present invention, the topical preparation is an ointment, patch, or gel, particularly an ointment.

Further, the drug may be for human or veterinary use.

Further, the drug may be used alone or in combination with other kinds of active ingredients.

In a second aspect of the present invention, provided is a topical preparation for treating and/or preventing psoriasis diseases, the topical preparation including the compound described in the first aspect, or a pharmaceutically acceptable salt, stereoisomer, isotope derivative, solvate (such as hydrate), prodrug, metal chelate, crystal form, or metabolite thereof;
preferably, the topical preparation may also include a pharmaceutically acceptable excipient.

Further, the topical preparation is selected from an aqueous solution, tincture, spirit, paste, oil, ointment, water-in-oil cream, salve, oil-in-water cream, gel, film forming agent, paint, liniment, lotions, spray, suspension, solution, emulsion, microemulsion, suspension, plaster, gel, patch, powder aerosol, suppository, eye drops, or nasal drops;
preferably, the excipients are of pharmaceutical grade.

More preferably, the topical preparation is an ointment, patch, or gel.

In some embodiments of the present invention, the topical preparation is an ointment or patch, particularly an ointment.

Further, the excipient includes one or more of a carrier, vehicle, diluent, wetting agent, filler, binder, lubricant, disintegrant, antioxidant, buffer, suspending aid, solubilizer, thickening agent, stabilizer, flavoring agent, preservative, and the like.

Further, the excipient includes one or more of a solubilizer, oil phase, aqueous phase, and additive.

Preferably, the solubilizer includes one or more of fatty alcohol polyoxyethylene ether, fatty acid polyoxyethylene ester, castor oil polyoxyethylene ether, alkylphenol polyoxyethylene ether, fatty amine polyoxyethylene ether, polyoxyethylene polyoxypropylene copolymer, polyethylene glycol, polyethylene glycol stearate, sorbitol ester polyoxyethylene ether, sorbitan ester.

Preferably, the oil phase includes one or more of liquid paraffin, solid paraffin, stearic acid, white petrolatum, hexadecanol, octadecanol, glyceryl monostearate, isopropyl myristate, dimethicone, silicone, beeswax, lanolin, spermaceti, caprylic/capric triglyceride, isooctyl palmitate, isopropyl palmitate, PEG-100 stearate, vegetable oil, animal oil.

Preferably, the aqueous phase includes one or more of water, ethanol, glycerol, hexadecanol, octadecanol, hexylene glycol, propylene glycol, propanetriol, dipropylene glycol, butylene glycol, pentylene glycol.

Preferably, the additive includes one or more of an emulsifier, transdermal penetration enhancer, pH adjuster, preservative.

More preferably, the transdermal penetration enhancer is one or more of azone, dimethylsulfoxide, decylmethylsulfoxide, menthol, borneol, clove oil, ethanol, laurel oil, urea, capsaicin, oleic acid, urea.

More preferably, the pH adjusting agent includes one or more of citric acid, salicylic acid, boric acid, fatty acid, sodium hydroxide, potassium hydroxide.

More preferably, the emulsifier is selected from one or more of triethanolamine, fatty acid soap, sodium lauryl sulfate, polyoxyethylene ether, polyoxypropylene ether, ethylene oxide, lecithin, gum arabic.

More preferably, the preservative includes one or more of imidazolidinyl urea, diazoimidazolidinyl urea, DMDMH, quaternary ammonium salt-15, benzoic acid preservative and derivatives thereof, phenoxyethanol, benzyl alcohol, polyhydric alcohol preservatives, chlorphenesin, Bronopol, Kathon, benzalkonium bromide, benzalkonium chloride, ethylparaben, methylparaben, isopropanol, salicylic acid, sorbic acid.

And/or the additive further includes one or more of vitamin E, dextran, betaine, butylated hydroxyanisole, ascorbic acid, iminodipropionic acid, ethanol, benzalkonium bromide, and hydroxypropanamide benzoic acid.

Further, when the drug is in the form of a gel, the excipient further includes one or more of gelatin, carbomer, methyl cellulose, sodium carboxymethyl cellulose, hydroxypropyl methyl cellulose, polyvinylpyrrolidone, polyvinyl alcohol, sodium alginate, glycerol, propylene glycol, dipropylene glycol, water.

Further, the topical preparation is preferably in a unit dosage form. In such form, the preparation is subdivided into unit doses containing appropriate quantities of the active ingredient. The unit dosage form may be an ointment, patch, or any other dosage form; alternatively, the unit dosage form can be a packaged preparation, such as an ointment or patch packaged in a vial or box.

Further, the topical preparation may be used alone or in combination with other kinds of active ingredients.

Further, the quantity of active ingredients in the unit dosage form may be varied or adjusted from 10 ng to 10 mg (in particular, 10 ng, 20 ng, 50 ng, 100 ng, 200 ng, 500 ng, 1 µg, 2 µg, 5 µg, 10 µg, 20 µg, 50 µg, 100 µg, 200 µg, 500 µg, 1 mg, 2 mg, 5 mg, or 10 mg) according to the particular application and the potency of the active ingredient and, if desired, the preparation can also include other suitable therapeutic agents.

Preferably, the quantity of active ingredients in the unit dosage form of preparation is from 10 µg to 10 mg.

The various dosage forms of the drug described in the present invention can be prepared according to conventional production methods in the pharmaceutical field.

Further, the topical preparation may include 0.01-99.5% by weight (specifically, 0.01%, 0.02%, 0.025%, 0.03%, 0.04%, 0.05%, 0.06%, 0.07%, 0.08%, 0.09%, 0.1%, 0.12%, 0.14%, 0.16%, 0.18%, 0.2%, 0.22%, 0.24%, 0.26%, 0.28%, 0.3%, 0.32%, 0.34%, 0.36%, 0.38%, 0.4%, 0.42%, 0.44%, 0.46%, 0.48%, 0.5%, 0.52%, 0.54%, 0.56%, 0.58%, 0.6%, 0.62%, 0.64%, 0.66%, 0.68%, 0.7%, 0.75%, 0.8%, 0.85%, 0.9%, 0.95%, 1%, 2%, 3%, 4%, 5%, 6%, 7%, 8%, 9%, 10%, 20%, 30%, 40%, 50%, 60%, 70%, 80%, 90%, 95%, 99%, 99.5%), preferably 0.02-0.5%, more preferably 0.1%-0.5%, of the compound according to the first and/or second aspect of the present invention, or a pharmaceutically acceptable salt, stereoisomer, isotopic derivative, solvate (e.g. hydrate), prodrug, metal chelate, crystalline form, or metabolite thereof.

Further, the topical preparation may be for human or veterinary use.

In a specific embodiment of the present invention, the topical preparation includes: the compound according to the first and/or second aspect of the present invention, or a pharmaceutically acceptable salt, stereoisomer, isotopic derivative, solvate (e.g. hydrate), prodrug, metal chelate, crystalline form or metabolite thereof, oil phase, aqueous phase, additive;
the oil phase includes paraffin, glycerol monostearate, white petrolatum, and lanolin;
the aqueous phase includes propanetriol, water; and
the additive includes an emulsifier and a preservative;

In a specific embodiment of the present invention, the preservative is ethylparaben.

In a specific embodiment of the present invention, the emulsifier is triethanolamine.

In a third aspect of the present invention, provided is a method for preparing the aforementioned topical preparation, the method including the following steps: (1) taking an aqueous phase, a preservative, and an emulsifier, mixing, heating, and then adding an active ingredient, which is the compound according to the first and/or second aspects of the present invention, or a pharmaceutically acceptable salt, stereoisomer, isotopic derivative, solvate (e.g. hydrate), prodrug, metal chelate, crystalline form, or metabolite thereof, and stirring until dissolved to obtain an aqueous mixture; and
(2) taking the oil phase, mixing, and heating the components, stirring until dissolved, adding the aqueous phase mixture obtained in step (1) to the oil phase, and condensing into an ointment form while stirring.

Further, the oil phase, the aqueous phase, the preservative, and the emulsifier have the definitions set forth in the third aspect of the present invention.

Further, in the oil phase, the mass ratio of liquid paraffin, glycerol monostearate, white petrolatum, and lanolin is 1-3 : 3-5 : 2-4 : 1-3.

In some embodiments of the present invention, the mass ratio of liquid paraffin, glyceryl monostearate, white petrolatum, and lanolin in the oil phase is 2 : 4 : 3 : 2.

Further, in the aqueous phase, the mass ratio of glycerol to water is 2-4 : 10-18.

In some embodiments of the present invention, in the aqueous phase, the mass ratio of glycerol to water is 3 : 14.11.

Further, the mass ratio of the glycerol, ethylparaben, water, triethanolamine, and the active ingredient is 2-4 : 0.05-0.13 : 10-18 : 0.1-0.4 : 0.01-0.05.

In some embodiments of the present invention, the mass ratio of the glycerol, ethylparaben, water, triethanolamine, and active ingredient is 3 : 0.09 : 14.11 : 0.3 : 0.0285.

Further, in step (1), the temperature of the heating is 50-100°C (specifically, 50, 55, 60, 65, 70, 75, 80, 85, 90, 95, 100°C), preferably 70-90°C, more preferably 75-80°C.

Further, in step (1), the stirring is performed at a rotation speed of 200-500 r/min (specifically, 200, 250, 300, 350, 400, 450, 500 r/min), preferably at 350 r/min.

Further, step (1) includes: taking an aqueous phase, preservative, and emulsifier, mixing, heating in a water bath, adding active ingredients when the temperature reaches 75-80°C, and stirring until dissolved to obtain an aqueous mixture.

Further, in step (2), the temperature of the heating is 50-100°C (specifically, 50, 55, 60, 65, 70, 75, 80, 85, 90, 95, 100°C), preferably 70-90°C, more preferably 80°C.

Further, in step (2), the stirring is performed at a rotation speed of 200-500 r/min (specifically, 200, 250, 300, 350, 400, 450, 500 r/min), preferably at 250 r/min.

Further, step (2) includes: taking each component of the oil phase, mixing, and heating to 80°C in a water bath, stirring until dissolved, slowly adding the aqueous phase mixture obtained in step (1) into the oil phase, placing it at room temperature after stirring, and then condensing into an ointment with stirring.

In a fourth aspect of the present invention, provided is a method for treating and/or preventing psoriasis-like disease, the method including administrating to a subject the compound described in the first aspect, or a pharmaceutically acceptable salt, stereoisomer, isotope derivative, solvate (such as hydrate), prodrug, metal chelate, crystal form, or metabolite thereof, or the topical preparation of the second aspect;

Further, the administration can be by a gastrointestinal (e.g. oral) or parenteral (e.g. intravenous, intramuscular, subcutaneous, intradermal, intraorgan, intranasal, intraocular, instillation, intracerebral, intrathecal, transdermal, intrarectal, etc.) route.

In some embodiments of the present invention, the administration may be by injection or transdermal administration (e.g. application or microneedle administration).

Further, the pharmaceutical or topical preparation is preferably in a unit dosage form. In such form, the preparation is subdivided into unit doses containing appropriate quantities of the active component. The unit dosage form can be a capsule, tablet, or any dosage form; alternatively, the unit dosage form can be a packaged preparation, such as a tablet, capsule, powder, etc. packaged in a vial or ampoule.

Further, the pharmaceutical or topical preparation may be used alone or in combination with other types of active ingredients.

Further, the quantity of active ingredients in the unit dosage form may be varied or adjusted from 10 ng to 10 mg (in particular, 10 ng, 20 ng, 50 ng, 100 ng, 200 ng, 500 ng, 1 µg, 2 µg, 5 µg, 10 µg, 20 µg, 50 µg, 100 µg, 200 µg, 500 µg, 1 mg, 2 mg, 5 mg, or 10 mg) according to the particular application and the potency of the active ingredient and, if desired, the pharmaceutical or topical preparation can also include other suitable therapeutic agents.

Preferably, the quantity of active ingredients in the unit dosage form of preparation is from 10 µg to 10 mg.

The present invention specifically has the following beneficial effects:
(1) It has been found that in the present invention, Compounds 1, 2, and 5, especially Compound 1 can be used to treat psoriasis-like disease.
(2) It has been found that in the present invention, under the condition of the same administration dosage, in treating psoriasis-like disease, the effect of administration by application is better than the effect of administration by injection.
(3) Compound 1 is a conventional antiarrhythmic agent, and it has been found in the present invention that not all compounds having antiarrhythmic function have the effect of treating psoriasis-like diseases. The compound of the present invention has no effect on the treatment of psoriasis compared with the Flecainide ointment having a similar antiarrhythmic function, but the therapeutic effect of the compound 1 (i.e. propafenone) ointment of the present invention is particularly prominent.
(4) It has been found experimentally that in the present invention, Compound 3 (i.e. R-propafenone) has a better therapeutic effect on psoriasis-like diseases than Compound 1 (i.e. R/S-propafenone). From the above experimental results, the inventors speculated that, at the same mass concentration, the relative concentration of Compound 3 in Compound 1 was lower than that in Compound 3 alone because Compound 2 (i.e. S-propafenone) was contained in Compound 1, which may be the main reason why the therapeutic effect of Compound 1 was slightly lower than that of Compound 3.

As used herein, the terms "comprises", or "includes" are open-ended terms that encompass the specified ingredients or steps described, as well as additional specified ingredients or steps that do not materially affect the stated ingredients or steps.

As used herein, "treating" means slowing, interrupting, arresting, controlling, stopping, reducing, or reversing the progression or severity of a sign, symptom, disorder, condition, or disease after the disease has begun to progress, but does not necessarily involve complete elimination of all disease-related signs, symptoms, conditions, or disorders.

"Preventing" as used herein refers to a manner of preventing or delaying the onset of a disease or disorder or condition in a subject.

The "product" according to the present invention may be a medicament, a device or a kit or the like.

As used herein, "pharmaceutically acceptable" refers to a product that does not significantly stimulate an organism nor inhibit the biological activity and properties of the active substance of the product to which it is administered.

As used herein, "pharmaceutically acceptable salts" refer to salts prepared from pharmaceutically acceptable non-toxic acids or bases including inorganic acids or bases or organic acids or bases. The inorganic acid is selected from hydrochloric acid, hydrobromic acid, phosphoric acid, hydroiodic acid, or sulfuric acid. The inorganic base is selected from calcium, magnesium, lithium, sodium, zinc, aluminum, or potassium. The organic acid is selected from formic acid, glycolic acid, propionic acid, acetic acid, succinic acid, methanesulfonic acid, ethanesulfonic acid, cis-butenedioic acid, glutamic acid, benzoic acid, stearic acid, alginic acid, benzenesulfonic acid, glucuronic acid, pamoic acid, or galacturonic acid. The organic base is selected from diethanolamine, choline, procaine, lysine, or 1,2-ethylenediamine.

The "stereoisomer" used herein includes enantiomer, diastereomer, and geometric isomer forms.

A "hydrate" used herein refers to a compound that contains water, wherein the water may bind to the compound in a coordinate or covalent bond or hydrogen bond. For example, a compound crystallizes from an aqueous solution of its constituent ions, and the crystals that form are hydrates.

A "prodrug" used herein refers to a compound that has been chemically modified to render the drug inactive or less active at a non-specific target site *in vitro,* and is converted enzymatically or nonenzymatically *in vivo* to release the active drug, e.g. a compound of the present invention, into a pharmaceutically effective form.

A "solvate" used herein represents the physical combination of the compounds of the present invention with one or more solvent molecules. This physical association includes various degrees of ionic and covalent bonding, including hydrogen bonding. In some cases, the solvate may be isolated, such as when one or more solvent molecules are doped into the lattice of the crystalline solid. Solvate encompasses both solution-phase and an isolable solvate. Representative solvates include hydrates, ethanolates, and methanolates.

A "metabolite" used herein refers to a product of the chemical degradation of a compound of the present invention under physiological conditions in the body.

A "derivative" used herein represents a product formed by the substitution of an atom or group of atoms in a molecule of a compound with another atom or group of atoms, or a product formed by hydrolysis of a compound.

A "subject" used herein may be a human or non-human mammal, and the non-human mammal may be a wild animal, zoo animal, commercial animal, pet, laboratory animal, and the like. Preferably, such non-human mammals include but are not limited to, pigs, cows, sheep, horses, donkeys, foxes, raccoon dogs, mink, camels, dogs, cats, rabbits, mice (e.g. rats, mice, guinea pigs, hamsters, gerbils, dragon cats, squirrels) or monkeys, and the like.

"Intradermal injection" used herein means the injection of a drug into the skin below the epidermis, typically above the dermis, and not into the subcutaneous tissue.

An "alkyl" used herein refers to a straight or branched hydrocarbon chain radical that does not contain unsaturated bonds and is connected to other parts of the molecule by a single bond. Typical alkyl groups contain from 1 to 20 (e.g. 1, 2, 3, 4, 5, 6, 7, 8, 9, 10, 11, 12, 13, 14, 15, 16, 17, 18, 19, 20) carbon atoms, such as methyl, ethyl, n-propyl, isopropyl, n-butyl, sec-butyl, isobutyl, tert-butyl, n-pentyl, isopentyl, neopentyl, tert-pentyl, n-hexyl, isohexyl, n-heptyl, isoheptyl, n-octyl, nonyl, decyl, undecyl, 1-methylundecyl, dodecyl, tridecyl, tetradecyl, pentadecyl, hexadecyl, heptadecyl, octadecyl, nonadecyl, eicosyl, and the like. In the present invention, C₀ alkyl refers to H, i.e. C₀₋₁₀ alkyl (or C₀-C₁₀ alkyl) includes H and C₁₋₁₀ alkyl (or C₁-C₁₀ alkyl).

"Alkylene" used herein refers to a hydrocarbon radical (a divalent alkyl group) derived from the loss of two hydrogen atoms from an alkane molecule, which may be straight or branched and which is attached to the rest of the molecule by a single bond. Typical alkylene groups have 1 to 12 (e.g. 1, 2, 3, 4, 5, 6, 7, 8, 9, 10, 11, 12) carbon atoms, e.g. methylene (-CH2-), ethylene, propylene, butylene, and the like. In the present invention, C₀ alkylene refers to a single bond, i.e. C₀₋₁₀ alkylene (or C₀-C₁₀ alkylene) includes a single bond and C₁₋₁₀ alkylene (or C₁-C₁₀ alkylene).

"Alkoxy" used herein is composed of an alkyl group combined with an oxygen atom, for example, methoxy, ethoxy, propoxy, isopropoxy, n-butoxy, sec-butoxy, isobutoxy, tert-butoxy, 2-ethylbutoxy, n-pentoxy, isopentoxy, 1-methylpentoxy, 1, 3-dimethylbutoxy, n-hexoxy, 1-methylhexoxy, n-heptoxy, isoheptoxy, 1, 1, 3, 3-tetramethylbutoxy, 1-methylheptoxy, 3-methylheptoxy, n-octoxy, 2-ethylhexyloxy, 1, 1, 3-trimethylhexoxy, 1, 1, 3, 3-tetramethylpentyloxy, nonyloxy, decyloxy, undecyloxy, 1-methylundecyloxy, dodecyloxy, 1, 1, 3, 3, 5, 5-hexamethylhexyloxy, tridecyloxy, tetradecyloxy, pentadecyloxy, hexadecyloxy, heptadecyloxy, octadecyloxy, and eicosyloxy.

"Alkenyl" used herein refers to a straight or branched hydrocarbon chain radical containing at least two carbon atoms and at least one unsaturated bond and is attached to the rest of the molecule by a single bond. Typical alkenyl groups contain 2 to 12 (e.g. 2, 3, 4, 5, 6, 7, 8, 9, 10, 11, 12) carbon atoms, for example, ethenyl, 1-methyl-ethenyl, 1-propenyl, 2-propenyl or butenyl, and the like.

"Alkynyl" used herein refers to a straight or branched hydrocarbon chain radical containing at least two carbon atoms and at least one carbon-carbon triple bond and is attached to the rest of the molecule as a single bond. Typical alkynyl groups contain 2 to 12 (e.g. 2, 3, 4, 5, 6, 7, 8, 9, 10, 11, 12) carbon atoms, for example ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl, 1-methyl-3-butynyl, or 2-methyl-3-butynyl) and the like.

"Halogen atom" used herein includes fluorine, chlorine, bromine, and iodine.

"Cycloalkyl" used herein refers to alicyclic hydrocarbons, e.g. containing 1 to 4 monocyclic and/or fused rings, containing 3 to 18 carbon atoms, preferably 3 to 10 (e.g. 3, 4, 5, 6, 7, 8, 9, 10) carbon atoms, such as cyclopropyl, cyclobutyl, cyclopentyl, cyclohexyl, adamantyl, and the like.

"Haloalkyl" used herein refers to a group formed by the substitution of one or more hydrogens of an alkyl group with a halogen atom (e.g. fluorine, chlorine, bromine, or iodine), such as -CHF₂, -CH₂F, -CF₃, -CH₂-CF₃, -CH₂CH₂-CF₃, -CH₂CH₂CH₂-CF₃.

"Aryl" used herein refers to monocyclic or polycyclic radicals, including polycyclic radicals containing a monoaryl group and/or a fused aryl group, e.g. containing from 1-3 monocyclic or fused rings and 6-18 (e.g. 6, 8, 10, 12, 14, 16, 18) carbon ring atoms. As used herein, C₆-C₁₂ aryl refers to an aryl group containing 6-12 carbon ring atoms, e.g. phenyl, naphthyl, biphenyl, indenyl, and the like.

"Heterocyclyl" used herein refers to a 3- to 18-membered non-aromatic cyclic group including 2-17 carbon atoms and 1-10 heteroatoms. The heterocyclyl can be a monocyclic, bicyclic, tricyclic, or tetracyclic ring system, and can include fused spiro, or bridged ring systems. The heterocyclyl can be partially saturated (heteroaromatic) or fully saturated (heterocyclic alkyl). Suitable heteroaryl groups in the compounds of the present invention contain 1, 2, or 3 kinds of heteroatoms selected from N, O, S, or P atoms and include, for example, coumarin, including 8-coumarin, quinolinyl, including 8-quinolinyl, isoquinolinyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothienyl, benzothiazolyl, benzoxazolyl, quinazolinyl, quinoxalinyl, naphthyridinyl, furopyridinyl. Suitable heterocycloalkyl groups in the compounds of the present invention contain 1, 2, or 3 kinds of heteroatoms selected from N, O, or S atoms and include, for example, pyrrolidinyl, tetrahydrofuranyl, dihydrofuranyl, tetrahydrothienyl, tetrahydrothiopyranyl, piperidinyl, morpholinyl, thiomorpholinyl, oxathianyl, piperazinyl, azetidinyl, oxetanyl, thietanyl, azepanyl, oxiranyl, thietanyl, azepinyl, oxazepanyl, diazepinyl, triazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo [3.1.0] hexyl, 3-azabicyclo [4.1.0] heptyl, 3H-indolyl, and quinolizinyl. In the present invention, for an optionally substituted heterocyclyl group, the position of substitution may be on any suitable carbon atom or heteroatom. For example, for 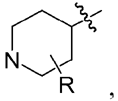 the position of substitution of R may be on any suitable carbon atom or nitrogen atom, which may be, for example,

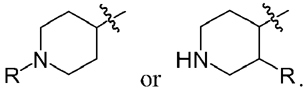

In the present invention, "D" refers to deuterium; "substituted with deuterium" refers to the replacement of one or more hydrogen atoms with a corresponding number of deuterium atoms.

It will be appreciated that, depending on the source of the chemical material used in the synthesis, there may be some variation in the natural isotopic abundance of the synthesized compound. Thus, the compounds of the present invention will inherently contain minor amounts of deuterated isotopologues. Despite this variation, the concentration of such naturally abundant stable hydrogen and carbon isotopes is low and inconsequential as compared to the degree of stable isotopic substitution of the compounds of the present invention. See, e.g. Wada, E. et al. Seikagaku, 1994, 66: 15; Gannes, LZ et al., Comp Biochem Physiol Mol Integr Physiol, 1998, 119: 725.

In the compounds of the present invention, any atom therein not designated as deuterium is present at its natural isotopic abundance. Unless otherwise indicated, when a position is designated specifically as "H" or "hydrogen", the position is understood to have hydrogen at its natural abundance isotopic composition. Likewise, unless otherwise indicated, when a position is specifically designated as "D" or "deuterium", the position is understood to have deuterium at an abundance that is at least 3000 times greater than the natural abundance of deuterium, which is 0.015% (i.e. at least 45% deuterium incorporation).

"Isotopic enrichment factor" used herein refers to the ratio between the isotopic abundance and the natural abundance of a specified isotope.

In other embodiments, a compound of the present invention has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

An "isotopologue" used herein refers to a substance wherein the chemical structure differs from a specified compound of the present invention only in its isotopic composition.

"Psoriasis-like disease" as used herein includes diseases such as psoriasis (e.g. psoriasis vulgaris, pustular psoriasis (e.g. Zumbusch, Barber, also known as palmoplantar pustular psoriasis), arthropathic psoriasis, erythrodermic psoriasis), parapsoriasis (e.g. parapsoriasis guttata, parapsoriasis plaque, parapsoriasis lichenoides, parapsoriasis varioliformis), pityriasis rubra pilaris, pityriasis rosea, lichen planus, lichen sclerosus, lichen pilaris, chronic eczema, parapsoriasis (such as guttata-type parapsoriasis, plaque-type parapsoriasis, licheniform parapsoriasis, acneiform parapsoriasis), or seborrheic dermatitis, and other diseases. In particular, the psoriasis-like disease described in the present invention is preferably psoriasis.

### DESCRIPTION OF THE DRAWINGS

Hereinafter, embodiments of the present invention will be described in detail with reference to the accompanying drawings.
FIG. 1: HPLC spectrogram before separation.
FIG. 2: HPLC spectrogram of Compound 2 Standard.
FIG. 3: HPLC spectrogram of Compound 3 Standard.
FIG. 4: Score results of different administration methods for Compound 1 in the treatment of psoriasis.
FIG. 5: Comparative study on the efficacy of Compound 1 and fluconazole in the treatment of psoriasis.
FIG. 6. Dose-effect experiments for the treatment of psoriasis by Compound 1.
FIG. 7: PASI score assessing the efficacy of Compounds 2 and 3 in treating psoriasis.
FIG. 8: PASI score assessing the efficacy of Compounds 4 and 5 in treating psoriasis.

### SPECIFIC IMPLEMENTATIONS

The technical solutions in the examples of the present invention will be described clearly and completely in conjunction with the accompanying drawings in the examples of the present invention. Obviously, the described embodiments are a part of the examples of the present invention, rather than all the examples. Based on the examples of the present invention, all other embodiments obtained by a person of ordinary skill in the art without inventive effort fall within the scope of the present invention.

Animals, sources of reagents used in the examples:
Mice used were purchased from Vital River, female, body weight 18-20 g;
Recombinant IL-23 was purchased from Novoprotein, Art.No. CS31;
Flecainide was purchased from MedChem Express, Art. No. HY-17429;
Compound 1, molecular formula C₂₁H₂₇NO₃, CAS No. 54063-53-5, molar mass: 341.444 g/mol, boiling point: 519.6°C, structural formula: 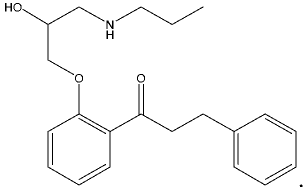
Compound 1 injection was purchased from Shanghai sine, Art. No. 0114006.
Compound 1 powder bulk drug was purchased from Henan Qixin, Art. No. H22091.
Compound 4-1, 4-2, 4-4, 5-4, and n-propylamine, purchased from Shanghai Bidepharm Co. Ltd. Article No: compound 4-1: BD20230110, compound 4-2: BD20221219, compound 4-4: BD20221118, compound 5-4: BD20230511, and n-propylamine: BD20231015.

### Example 1 Preparation of Compounds 2 and 3

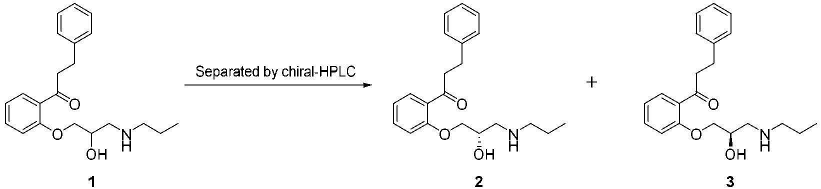

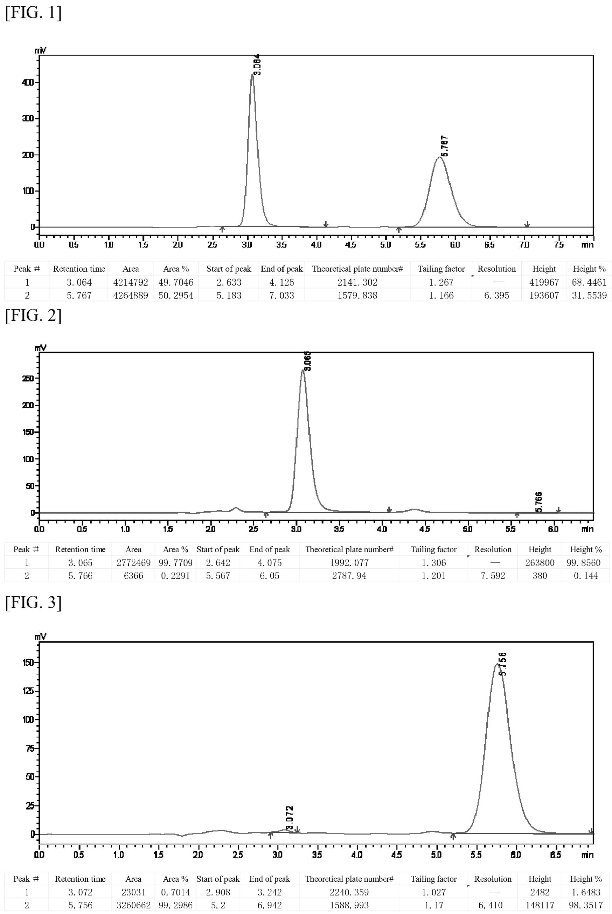

150 Mg of the free base of compound 1 (R/S-Propafenone) was dissolved in 2 mL of ethanol as a sample solution. Compound 1 was separated using high performance liquid chromatography and the results are shown in FIG. 1. The chromatogram shows high resolution between the two peaks. The solutions obtained with retention times of 3.1 min (peak 1) and 5.8 min (peak 2), respectively, were concentrated under reduced pressure to give 50 mg (peak 1) and 60 mg (peak 2) of a white powder. The compounds corresponding to Peak 1 and Peak 2 were characterized using HPLC and compared to the HPLC spectrograms of the Compound 2 standard and the Compound 3 standard (as shown in FIGS. 2-3). The results showed that Peak 1 was S-propafenone (i.e. Compound 2) and Peak 2 was R-propafenone (i.e. Compound 3).

The conditions for high-performance liquid chromatography are as follows:

Liquid chromatography instrument: K-Prep LAB 100G (YMC CO., LTD.); model of the column: CHIRALPAK AY; dimensions of the column: 2.5 cm I.D. x 25 cm L; specification of the filler: 10 µm; mobile phase: N-hexane: ethanol = 40 : 60; flow rate: 50 mL/min; detection wavelength: UV210 nm; and column temperature: 40°C.

### Example 2: Synthesis of Compound 4

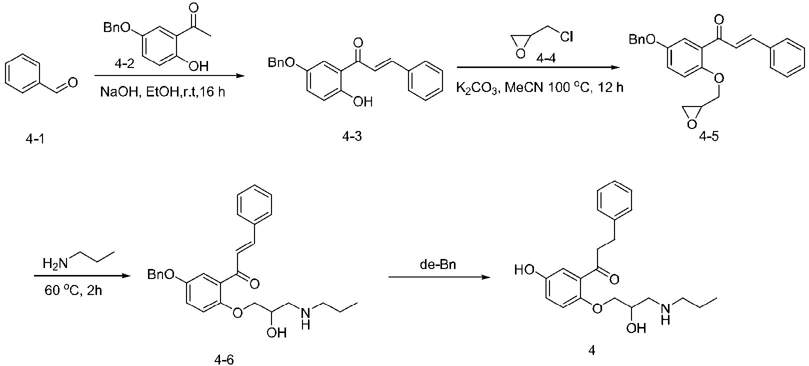

### (1) Synthesis of Compound 4-3

Compound 4-1 (876 mg, 8.26 mmol) was dissolved in EtOH (80 mL), to which Compound 4-2 (2.00 g, 8.26 mmol) was added, followed by dropwise addition of sodium hydroxide (1.32 g, 33.02 mmol) dissolved in water (13.2 mL), after which the reaction was stirred at 25°C for 16 hours. The reaction was monitored by LCMS for the target product. The reaction solution was poured into ice water, added with 1M hydrochloric acid solution slowly to pH = 4-5, and extracted with ethyl acetate. The organic phase was collected, dried over anhydrous sodium sulfate, compressed, and stirred with silica gel. The mixture was purified by passing through a Flash column machine (eluent: ethyl acetate: petroleum ether = 0-30%) to afford Compound 4-3 (1.8 g, yield: 46.2%, purity: 70%), whose structure was confirmed by LCMS.

LCMS: MS=331.1 [M+H]⁺.

### (2) Synthesis of Compound 4-5

Compound 4-3 (940 mg, 2.85 mmol) and Compound 4-4 (1.32 g, 14.23 mmol) were dissolved in a sealed jar containing acetonitrile (20 mL), to which was added potassium carbonate (786.5 mg, 5.69 mmol), after which the reaction was stirred at 100°C for 12 hours. The product was monitored by LCMS. The reaction mixture was poured into water and extracted with ethyl acetate. The organic phase was collected, washed with brine, dried over anhydrous sodium sulfate, compressed, and stirred with silica gel. The mixture was purified by passing through a Flash column machine (ethyl acetate: petroleum ether = 0-50%) to afford Compound 4-5 (330 mg, yield: 30%), whose structure was confirmed by LCMS.

LCMS: MS=387.1 [M+H]⁺.

### (3) Synthesis of Compound 4-6

Compound 4-5 (330 mg, 0.854 mmol) was added along with n-propylamine (101 mg, 1.71 mmol) to a sealed jar containing acetonitrile (6 mL) and stirred at 60°C for 2 hours. The product was monitored by LCMS and the reaction was passed directly through a Flash reverse-phase column to afford Compound 4-6 (160 mg, yield: 42%), whose structure was confirmed by LCMS.

LCMS: MS=446.3 [M+H]⁺.

### (4). Synthesis of Compound 4

Compound 4-6 (160 mg, 0.359 mmol) was dissolved in methanol (3 mL), to which 10% palladium on carbon (76 mg, 0.072 mmol) was added thereto, after which, the reaction system was replaced with hydrogen 3 times, and stirred under a hydrogen atmosphere for 1 hour. The reaction was monitored by LCMS for completion and filtered through a filter head. The filtrate was collected and, stirred with silica gel. The mixture was purified by passing through a Flash column filter (methanol: dichloromethane = 0 ~ 50%, 1% aqueous ammonia) to give Compound 4 (39 mg, yield: 30%), whose structure was confirmed by LCMS and HNMR.

LCMS:M=358.2 [M+H]⁺.

¹H NMR (400 MHz, DMSO) δ 7.29 - 7.20 (m, 4H), 7.17 (d, *J =* 6.8 Hz, 1H), 6.96 (dd, *J* = 15.8, 5.9 Hz, 2H), 6.90 (dd, *J =* 8.8, 3.1 Hz, 1H), 4.08 - 3.84 (m, 3H), 2.89 (t, *J =* 7.5 Hz, 2H), 2.68 (dt, *J =* 12.2, 6.7 Hz, 2H), 2.50 - 2.38 (m, 4H), 1.42 (dd, *J =* 14.6, 7.3 Hz, 2H), 1.23 (s, 2H), 0.84 (t, *J =* 7.4 Hz, 3H).

### Example 3: Synthesis of Compound 5

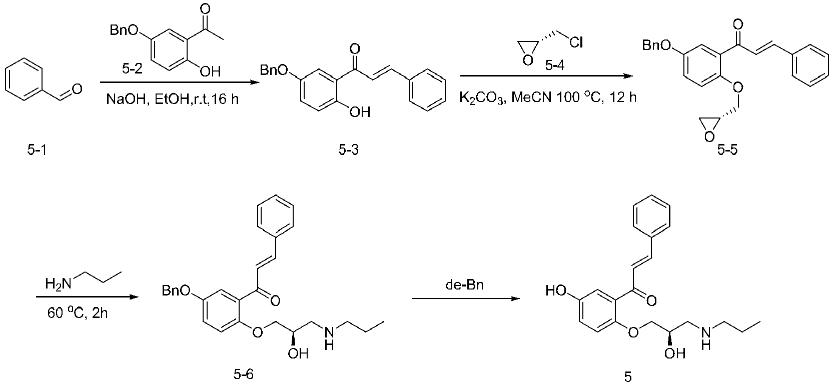

### (1) Synthesis of Compound 5-3

Compound 5-1 (876 mg, 8.26 mmol) was dissolved in ethanol (80 mL), to which Compound 5-2 (2.00 g, 8.26 mmol) was added, followed by dropwise addition of a solution of sodium hydroxide (1.32 g, 33.02 mmol) dissolved in water (13.2 mL), after which the reaction was stirred at 25°C for 16 hours. The reaction was monitored by LCMS for the target product. The reaction solution was poured into ice water, added with 1M hydrochloric acid solution slowly to pH = 4-5, and extracted with ethyl acetate. The organic phase was collected, dried over anhydrous sodium sulfate, compressed, and stirred with silica gel. The mixture was purified by passing an automated column chromatography separation instrument (eluent: ethyl acetate: petroleum ether = 0-30%) to afford Compound 5-1 (750 mg, yield: 27.5%), whose structure was confirmed by LCMS.

LCMS: MS=331.1 [M+H]⁺.

### (2) Synthesis of Compound 5-5

Compound 5-3 (400 mg, 1.21 mmol) and Compound 5-4 (118 mg, 4.84 mmol) were dissolved in a sealed reaction jar containing acetonitrile (10 mL), to which was added potassium carbonate (335 mg, 2.42 mmol), after which the reaction was stirred at 100°C for 12 hours. The presence of the product was monitored by LCMS. The reaction mixture was poured into water and extracted with ethyl acetate. The organic phase was collected, washed with brine, dried over anhydrous sodium sulfate, compressed, and stirred with silica gel. The mixture was purified by passing a Flash column machine (eluent: ethyl acetate: petroleum ether = 0-50%) to give Compound 5-5 (330 mg, yield: 70%), whose structure was confirmed by LCMS.

LCMS: M=387.2 [M+H]⁺.

### (3) Synthesis of Compound 5-6

Compound 5-5 (330 mg, 0.854 mmol) was added along with n-propylamine (101 mg, 1.71 mmol) to a sealed reaction jar containing acetonitrile (6 mL) and stirred at 60°C for 2 hours. The product was monitored by LCMS and the reaction was directly purified by passing a reverse phase column (packed with C18) to afford Compound 5-6 (160 mg, yield: 42%), whose structure was confirmed by LCMS.

LCMS: M=446.3 [M+H]⁺.

### (4). Synthesis of Compound 5

Compound 5-6 (160 mg, 0.359 mmol) was dissolved in MeOH (3 mL), to which 10% palladium on carbon (76 mg, 0.072 mmol) was added thereto, after which, the reaction system was replaced with hydrogen 3 times, and stirred under a hydrogen atmosphere condition for 1 hour. The reaction was monitored by LCMS for completion and filtered through a filter head. The filtrate was collected and, stirred with silica gel. The mixture was purified by passing through a Flash column filter (eluent: MeOH: DCM=0-50%, 1% aqueous ammonia) to give Compound 5 (27.24 mg, yield: 21%), whose structure was confirmed by LCMS and HNMR.

LCMS: M=358.2 [M+H]⁺.

¹H NMR (400 MHz, CDCl₃) δ 7.19 (t, *J =* 6.5 Hz, 4H), 7.12 (d, *J* = 7.4 Hz, 1H), 6.98 (s, 1H), 6.71 (d, *J =* 8.5 Hz, 1H), 6.62 (d, *J =* 8.7 Hz, 1H), 3.90 (t, *J =* 24.6 Hz, 3H), 3.19 (s, 2H), 2.94 (t, *J =* 7.2 Hz, 2H), 2.80 - 2.16 (m, 4H), 1.47 (dd, *J =* 14.3, 6.9 Hz, 2H), 1.19 (s, 2H), 0.85 (t, *J* = 7.3 Hz, 3H).

### Example 4 Preparation of Compound 1 ointment

The formulation of Compound 1 in an ointment form included:
Oil phase: liquid paraffin 2 g, glycerol monostearate 4 g, white petrolatum 3 g, and lanolin 2 g;
Aqueous phase: propanetriol 3 g, and ultrapure water 14.11 g;
Main drug: compound 1 0.0285 g (Drug loading: 0.1%);
Emulsifier: triethanolamine 0.3 g; and
Preservative: ethylparaben 0.09 g.

Preparation method: the aqueous phase, preservative, and emulsifier were weighed into a 100 mL beaker, placed into a 500 mL beaker with a magnetic stirrer, and heated in a water bath. When the temperature reached 75°C, Compound 1 powder was added and stirred until dissolved (80°C, 350 r/min). The oil phase was weighed into another 100 mL beaker according to the formulation, heated in a water bath, and stirred until dissolved (80°C, 250 r/min). The aqueous phase was slowly added to the oil phase, stirred for 10 min (80°C, 300 r/min), then removed, placed at room temperature, and continuously stirred in the same direction until it condensed into a cream. The prepared ointment was placed in a 50 mL centrifuge tube and sealed with a sealing film.

The mass concentration of Compound 1 in the ointment was 0.1%.

### Example 5 Treatment of psoriasis with the compound of Formula I

### 1. Construction of mouse model of psoriasis

Psoriasis mouse model was established by intradermal injection of recombinant IL-23 (1 ng/20 µL PBS, per mouse per day for 4 consecutive days) into depilated mice on the back.

### 2. Test procedures

On Day 2 after modeling, the psoriasis model mice were randomly divided into 3 groups, with the same number of mice in each group, and divided into a model group, treatment group (the treatment group included a subcutaneous treatment group and an intradermal treatment group), with the model group as a control. Starting on Day 2, the mice in the subcutaneous treatment group were subcutaneously injected with compound 1 on the back at a dose of 0.07 mg per mouse per day, so as to simulate the effect of injection administration on psoriasis in mice. The mice in the intradermal treatment group were intradermally injected with Compound 1 on the back of mice at a dose of 0.07 mg per mouse per day to simulate the therapeutic effect of an application administration on psoriasis in mice. The model group was injected with 20 µL of normal saline intradermally. All three groups were injected for 4 consecutive days. On Day 6, the symptoms of the model and treatment groups were scored.

The calculation rule for the number of days was: the number of days will be calculated from the end of modeling, which will be recorded as Day 0, followed by Day 1, Day 2, etc.

The concentration of Compound 1 in the subcutaneous treatment group and the intradermal treatment group injection was 35 mg/10mL.

Scoring criteria: 0 points, no symptom; 1 point, skin erythema; 2 points, scattered scurf; 3 points, diffuse dandruff; 4 points, skin sclerosis; 5 points, erythroderma or death.

### 3. Test results

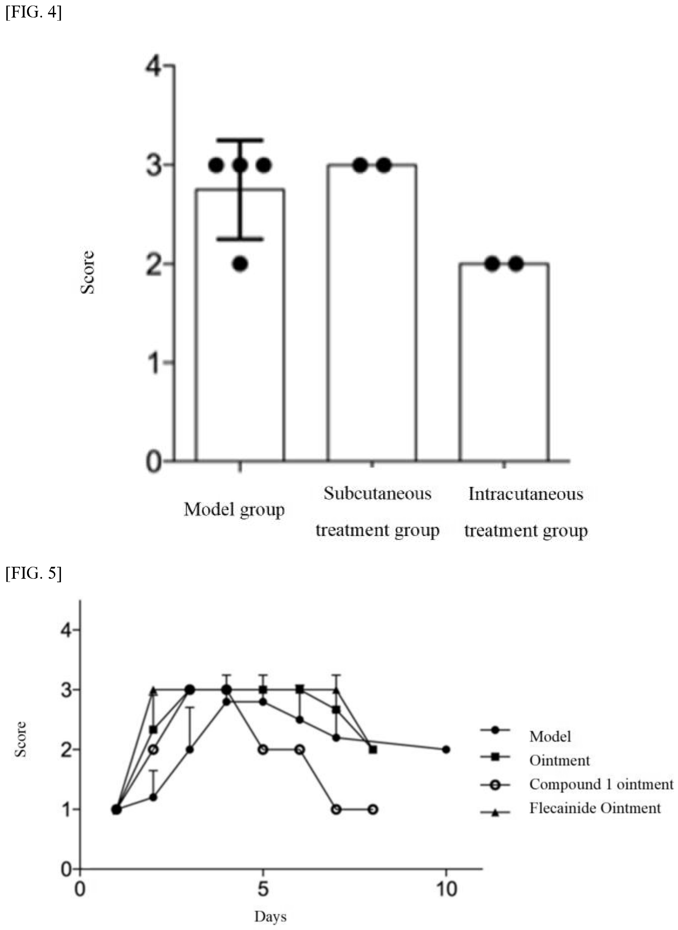

Results are as shown in FIG. 4, the scores of the model group and the subcutaneous treatment group were close to 3 points, and the scores of the intradermal treatment group were close to 2 points, indicating that the intradermal treatment group injected with Compound 1 injection had a significant therapeutic effect compared with the model group and the subcutaneous treatment group. Thus, it can be demonstrated that the administration of Compound 1 by application is superior to the administration by injection for treating psoriasis.

Dosing explorations showed that Compound 1 at 10 µg, 20 µg, 50 µg, 100 µg, 200 µg, 500 µg, 1 mg, 2 mg, 5 mg, and 10 mg intracutaneous injections had therapeutic effects.

### Example 6 Comparative efficacy studies with flecainide

### 1. Construction of mouse model of psoriasis

Same as Example 5.

### 2. Test procedures

Compound 1 and Flecainide are the same kind of conventional antiarrhythmic drugs, but the target sites and modes of action are different. In order to explore whether antiarrhythmic drugs all have the effect of treating psoriasis, this experiment compared the effect of Compound 1 and Flecainide in a mouse disease model.

On Day 2 after modeling, the psoriasis model mice were divided into 4 groups on average: the model group, the ointment group, the compound 1 ointment group, and the flecainide ointment group. Each group was applied with different substances (as shown in Table 1) on the backs of the mice for 4 consecutive days. The symptoms of the model mice and the treatment group mice were scored from Day 1.

**Table 1 Substances and doses applied to the backs of mice in each group**

| Grouping | Model group | Ointment group | Compound 1 ointment group | Flecainide ointment group |
|---|---|---|---|---|
| Applied substance | water | Ointment | Compound 1 ointment | Flecainide ointment |
| Application amount | 30 mg | 30 mg | 30 mg | 30 mg |

The calculation rule for the number of days was: the number of days will be calculated from the end of modeling, which will be recorded as Day 0, followed by Day 1, Day 2, etc.

The formulation and preparation method of the ointment group can be referred to as Example 4, which differed from Example 4 only in that the main drug was not added;

The formulation and preparation method of the Compound 1 ointment group can be referred to as Example 4 (wherein the mass concentration of Compound 1 in the Compound 1 ointment was 0.1%);

The formulation and preparation method of Flecainide ointment can be referred to as Example 4, which differed from Example 4 only in that the main drug was replaced with an equivalent amount of Flecainide (wherein the mass concentration of flecainide in the flecainide ointment was 0.1%).

Scoring criteria: 0 points, no symptom; 1 point, skin erythema; 2 points, scattered scurf; 3 points, diffuse dandruff; 4 points, skin sclerosis; 5 points, erythroderma or death.

### 3. Test results

The results are shown in FIG. 5. During the period from Day 1 to Day 5, the Flecainide Ointment group reached a score of 3 on Day 2 and then remained at a score of 3 on Days 2 to 7. The Compound 1 ointment group reached a score of 2 on Day 2, and a score of 3 on Days 3 to 4, and decreased from Day 5 to a score of 2 and below, and decreased to a score of 1 on Days 7 and 8. The above data show that the therapeutic effect of Compound 1 is significantly stronger than that of Flecainide at the same dose.

Overall, the application of Compound 1 ointment had a significant therapeutic effect compared to the model group, and neither the Flecainide ointment nor the ointment alone had a therapeutic effect, indicating that Compound 1 has a significant therapeutic effect in treating psoriasis compared to Flecainide.

### Example 7: dose-response relationship of compound 1

### 1. Construction of mouse model of psoriasis

Same as Example 5.

### 2. Test procedures

On Day 2 after modeling, the psoriasis model mice were divided into 7 groups: model group, ointment alone group, and 5 groups of different doses of treatment group. The model group served as the control group. Different substances were applied on the back of each group of mice (as shown in Table 2) for 4 consecutive days. On Day 6, the symptoms of the model mice and the treated mice were scored.

**Table 2 Substances and doses applied to the backs of mice in each group**

| | Mode 1 group | Ointment alone group | 1% Compound 1 ointment group | 0.5% Compound 1 ointment group | 0.2% Compound 1 ointment group | 0.1% Compound 1 ointment group | 0.01% Compound 1 ointment group |
|---|---|---|---|---|---|---|---|
| Applied substance | water | Ointment | Compound 1 ointment | Compound 1 ointment | Compound 1 ointment | Compound 1 ointment | Compound 1 ointment |
| Application amount | 30 mg | 30 mg | 30 mg | 30 mg | 30 mg | 30 mg | 30 mg |

The calculation rule for the number of days was: the number of days will be calculated from the end of modeling, which will be recorded as Day 0, followed by Day 1, Day 2, etc.

The formulation and preparation method of the ointment alone group can be referred to as Example 4, which differed from Example 4 only in that the main drug was not added;

Formulation and preparation methods of 0.01%, 0.1%, 0.2%, 0.5%, and 1% Compound 1 ointment can be referred to Example 4. The masses of the main drugs in the formulation of Example 4 can be adjusted to prepare Compound 1 ointment with mass fractions of 0.01%, 0.1 %, 0.2%, 0.5%, and 1% respectively.

Scoring criteria: 0 points, no symptom; 1 point, skin erythema; 2 points, scattered scurf; 3 points, diffuse dandruff; 4 points, skin sclerosis; 5 points, erythroderma or death.

### 3. Test results

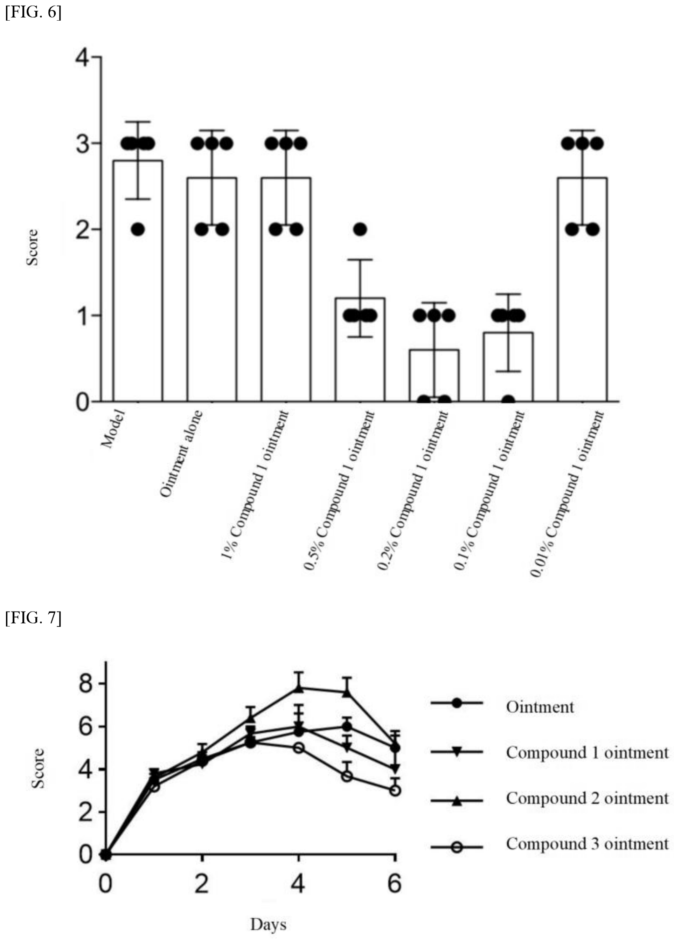

The results are shown in FIG. 6. The effective window dose of Compound 1 ointment was 0.1%-0.5% compared to the model group. In more detailed dose exploration, it was found that 0.02%, 0.025%, 0.03%, 0.04%, 0.05%, 0.06%, 0.07%, 0.08%, 0.09%, and 0.1% were still effective, indicating that when the mass concentration of Compound 1 in the Compound 1 Ointment was 0.02%-0.5%, Compound 1 ointment had significant therapeutic effect.

### Example 8 Evaluation of the efficacy of Compounds 2 and 3

### 1. Preparation of Compound 2 and 3 ointments

Same as Example 4.

### 2. Construction of mouse model of psoriasis

Same as Example 5.

### 3. Test procedures

On Day 2 after modeling, the psoriasis model mice were randomly divided into: the ointment group, the compound 1 ointment treatment group, the compound 2 ointment treatment group, and the compound 3 ointment treatment group. Each group was applied with different substances (as shown in Table 3) on the backs of the mice for 6 consecutive days. From Day 1, the symptoms of the back skin of the model mice and the mice in the treatment group were scored every day.

**Table 3 Substances and doses applied to the backs of mice in each group**

| | Ointment group | Compound 1 ointment treatment group | Compound 2 ointment treatment group | Compound 3 ointment treatment group |
|---|---|---|---|---|
| Applied substance | Ointment | Ointment | Ointment | Ointment |
| Application amount | 200 mg | 200 mg | 200 mg | 200 mg |

The calculation rule for the number of days was: the number of days will be calculated from the end of modeling, which will be recorded as Day 0, followed by Day 1, Day 2, etc.

The formulation and preparation method of the ointment group can be referred to as Example 4, which differed from Example 4 only in that the main drug was not added;

The formulation and preparation method of Compound 1 ointment group, Compound 2 ointment group, and Compound 3 ointment can be referred to as Example 4 (mass concentration of 0.025% each). Scoring criteria: 0 points, no symptom; 1 point, skin erythema; 2 points, scattered scurf; 3 points, diffuse dandruff; 4 points, skin sclerosis; 5 points, erythroderma or death.

### 4. Test results

PASI score results are shown in FIG. 7. The skin scores of mice in each group showed a typical incidence trend of psoriasis which increased first and then decreased with the lapse of time. At the end of the experiment, compared with the ointment group, the skin scores of Compound 1 and Compound 3 ointment treatment were significantly decreased, the symptoms were effectively alleviated, and the score of Compound 3 was lower, demonstrating that the treatment effect was more obvious, while the Compound 2 ointment showed no treatment effect. Since both Compound 3 and Compound 2 were isolated from Compound 1, Compound 2 had no therapeutic effect and Compound 3 had a therapeutic effect from the viewpoint of therapeutic results, which proved that Compound 3 was an active ingredient of Compound 1. At the same mass concentration, the relative concentration of Compound 3 in Compound 1 was lower than that in Compound 3 alone due to the presence of Compound 2 in Compound 1, which may be the main reason for the slightly lower therapeutic effect of Compound 1 than that in Compound 3 ointment group.

### Example 9 Evaluation of the efficacy of compounds 4 and 5

### 1. Construction of mouse model of psoriasis

Same as Example 5.

### 2. Test procedures

For compounds 4 and 5, due to poor transdermal effect, we switched to microneedle administration (MN) to verify its therapeutic effect. On Day 2 after modeling, the psoriasis model mice were randomly divided into a model group, a Compound 4 microneedle treatment group, and a Compound 5 microneedle treatment group on average. After the skin of the mice was broken with microneedles, 100 µL of 4/5 drops of the compound was put on the back of the mice with a pipette and gently Applied evenly (as shown in Table 3) for 8 consecutive days. The symptoms of the back skin of the model mice and the treatment group mice were scored every day starting from Day 1.

**Table 4 Types and doses of administration on the back of mice in each group**

| | Model group (microneedle) | Compound 4 (microneedle) | Compound 5 (microneedle) |
|---|---|---|---|
| Applied substance | Solvent | Solution | Solution |
| Application amount | 100 µL | 100 µL | 100 µL |

The calculation rule for the number of days was: the number of days will be calculated from the end of modeling, which will be recorded as Day 0, followed by Day 1, Day 2, etc.

Scoring criteria: 0 points, no symptom; 1 point, skin erythema; 2 points, scattered scurf; 3 points, diffuse dandruff; 4 points, skin sclerosis; 5 points, erythroderma or death.

### 3. Test results

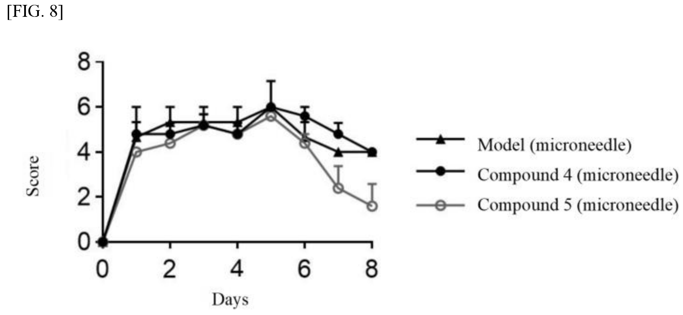

The results of PASI scores are shown in FIG. 8. The skin scores of mice in each group showed a typical incidence trend of psoriasis, which increased first and then decreased with the lapse of time, but the scores of each group were different. After Compound 5 treatment, skin scores decreased rapidly from Day 5 of treatment, and symptoms were effectively alleviated, compared to the microneedle-only treated model group.

The preferred embodiments of the present invention have been described in detail above, but the present invention is not limited to the specific details in the embodiments. Within the scope of the technical concept of the present invention, various simple modifications can be made to the technical solution of the present invention, all of which are within the scope of the present invention.

It should further be noted that the particular features described in the above-detailed description may be combined in any suitable manner without departing from the scope of the present invention and that the present invention may be practiced otherwise than as specifically described herein to avoid unnecessarily obscuring the present invention.

## Claims

1. Use of a compound represented by formula I or a pharmaceutically acceptable salt, stereoisomer, isotope derivative, solvate (such as hydrate), prodrug, metal chelate, crystal form, or metabolite thereof in the preparation of a drug for treating and/or preventing psoriasis-like disease, the compound having the following structure: 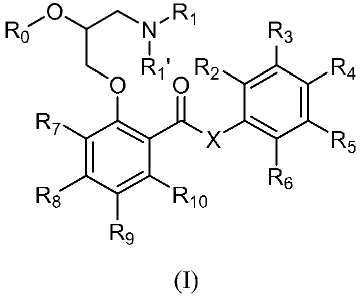
wherein X is selected from a single bond, -O-(C₀-C₁₀ alkylene)-, -S-(C₀-C₁₀ alkylene)-, -N(C₀-C₁₀ alkyl)-(C₀-C₁₀ alkylene)-, C₁-C₁₀ alkylene, C₂-C₁₀ alkenylene, C₂-C₁₀ alkynylene;
R₀ is selected from H, -COCC₁₋₁₀ alkyl), -CO(C₆₋₁₀ aryl);
R₁ and R₁' are independently selected from H, C₁-C₁₀ alkyl, C₂-C₁₀ alkenyl, C₂-C₁₀ alkynyl, C₁-C₁₀ haloalkyl, -(C₀-C₆ alkylene)-(C₃-C₁₀ cycloalkyl), -(C₀-C₆ alkylene)-(C₆-C₁₀ aryl), -(C₀-C₆ alkylene)-(4-10 membered heterocyclyl), -N(C₀₋₁₀ alkyl) (C₀₋₁₀ alkyl), -O(C₀₋₁₀ alkyl); or R₁ and R₁' together with the nitrogen atom to which they are attached form a heterocyclic ring, wherein H on the heterocyclic ring is optionally substituted by one or more groups selected from halogen, cyano, nitro, azido, C₁-C₁₀ alkyl, C₁-C₁₀ haloalkyl, -N(C₀₋₁₀ alkyl) (C₀₋₁₀ alkyl), -O(C₀₋₁₀ alkyl), -S(C₀₋₁₀ alkyl);
R₂, R₃, R₄, R₅, R₆, R₇, R₈, R₉, and R₁₀ are independently selected from H, halogen, cyano, nitro, azido, C₁-C₁₀ alkyl, C₁-C₁₀ haloalkyl, -(C₀-C₆ alkylene)-(C₃-C₁₀ cycloalkyl), -(C₀-C₆ alkylene)-(C₆-C₁₀ aryl), -(C₀-C₆ alkylene)-(4-10 membered heterocyclyl), -N(C₀₋₁₀ alkyl) (C₀₋₁₀ alkyl), -O (C₀₋₁₀ alkyl), -S (C₀₋₁₀ alkyl);
preferably, X is selected from C₁-C₆ alkylene, C₂-C₆ alkenylene,
more preferably -CH₂CH₂- or -CH = CH-;
preferably, R₀ is selected from H, -CO(C₁₋₆ alkyl);
Preferably, R₁ and R₁' are independently selected from: H, C₁-C₆ alkyl, -(C₀-C₆ alkylene)-(C₆-C₁₀ aryl); or R₁ and R₁' together with the nitrogen atom to which they
are attached form a 4-6 membered nitrogen-containing heterocyclic ring, wherein H on the 4-6 membered nitrogen-containing heterocyclic ring is optionally substituted with one or more groups selected from C₁-C₆ alkyl; and
preferably, R₂, R₃, R₄, R₅, R₆, R₇, R₈, R₉, and R₁₀ are independently selected from H, halogen, C₁-C₆ alkyl, C₁-C₆ haloalkyl, -O(C₀₋₆ alkyl).

2. The use of claim 1, wherein the compound has the following structure: 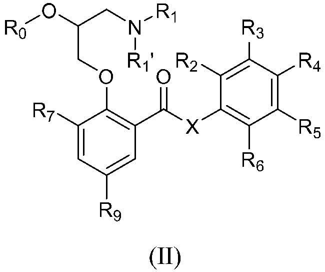
preferably, the compound has the following structure: 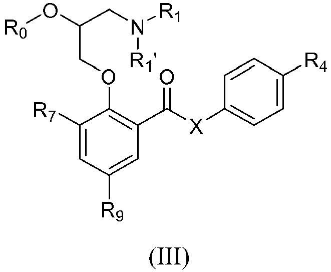
more preferably, R₄ is H, F, Cl, Br, I, or -CF₃; and
more preferably, R₇ or R₉ is H or -OH.

3. The use of claim 1, wherein the compound has the following structure: 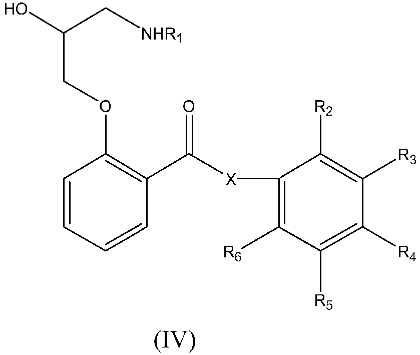
wherein X is selected from a single bond, -O-, -S-, or alkylene;
R₁ is selected from H, alkyl, alkoxy, alkenyl, or alkynyl;
R₂, R₃, R₄, R₅, and R₆ are independently selected from H, alkyl or halogen atoms.

4. The use of claim 3, wherein X is selected from a single bond or C₁-C₁₀ alkylene.

5. The use of claim 3 or 4, wherein R₁ is selected from a C₁-C₁₀ alkyl.

6. The use of any one of claims 3-5, wherein R₂, R₃, R₄, R₅, and R₆ are independently selected from H, F, Cl, Br, I, methyl, or ethyl.

7. The use of any one of claims 3-6, wherein
X is selected from C₁-C₄ alkylene;
R₁ is selected from a C₁-C₄ alkyl;
R₂, R₃, R₄, R₅, and R₆ are independently selected from H, F, Cl, Br, I, methyl, or ethyl.

8. The use of any one of claims 3-7, wherein the compound has the following structure:

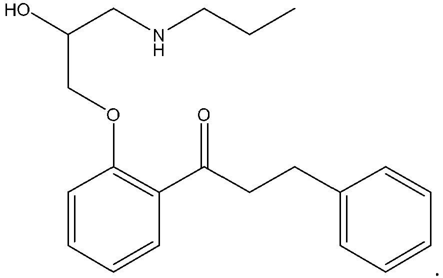

9. The use of claim 8, wherein the stereoisomer of the compound has the following structures: 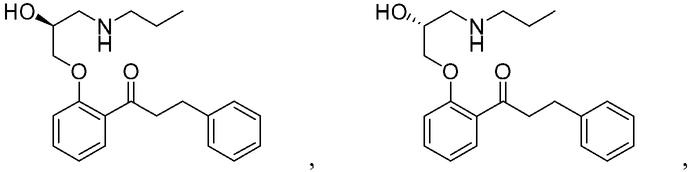 preferably

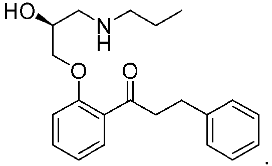

10. The use of claim 1, wherein the compound has the following structures: 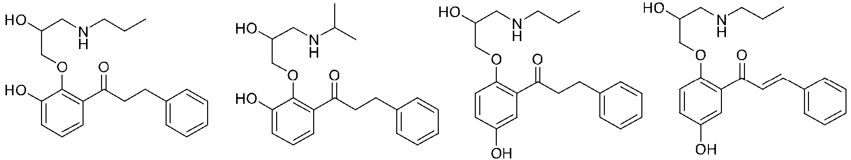 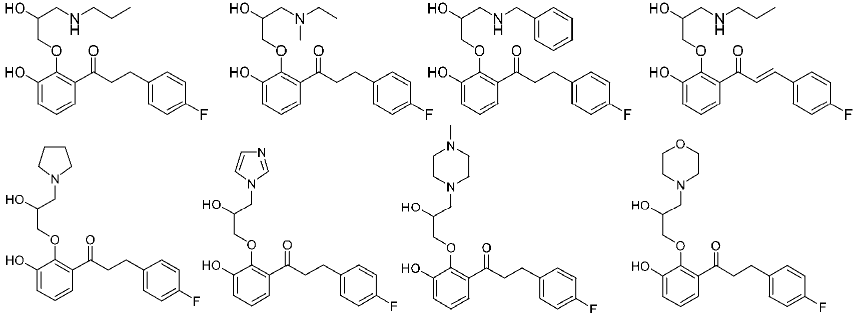 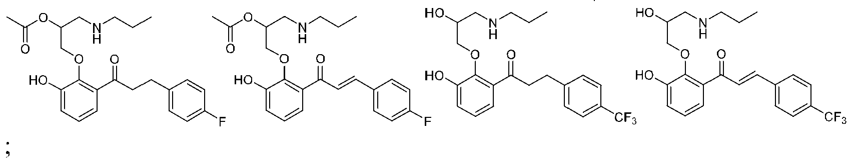
preferably, the stereoisomer has the following structures: 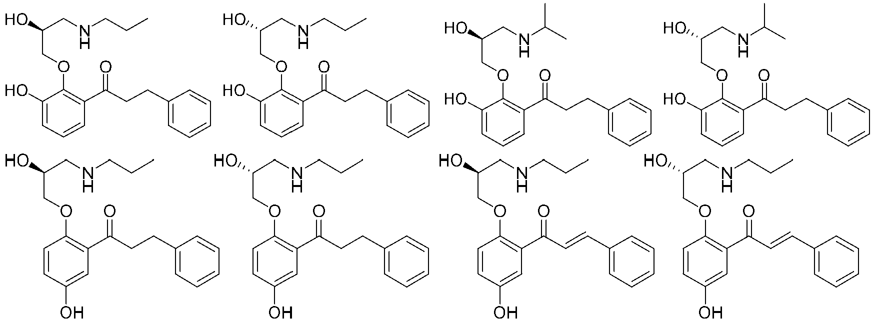 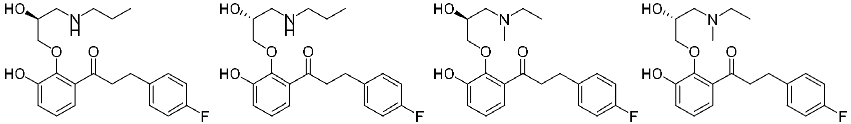 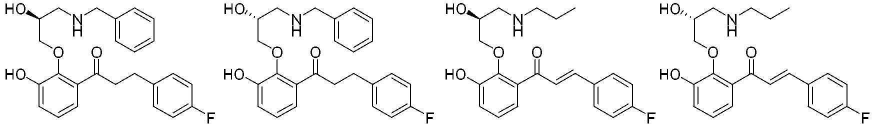 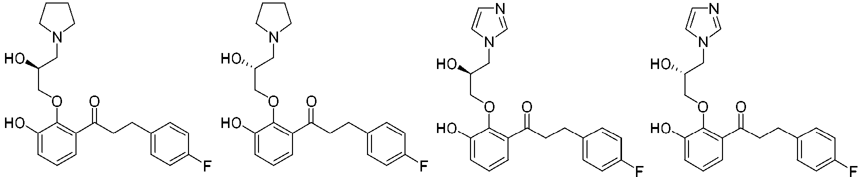 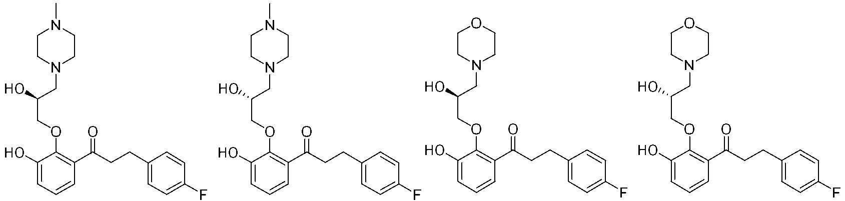 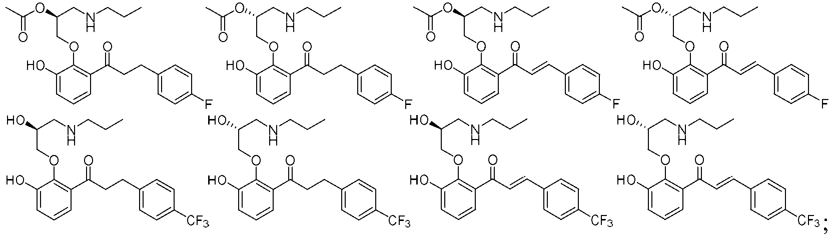
more preferably, the stereoisomer has the following structures: 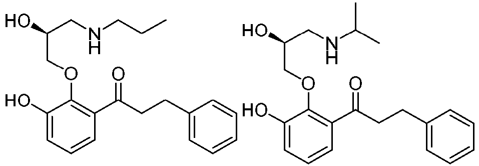 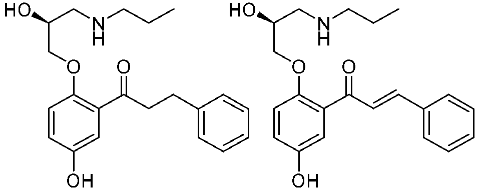 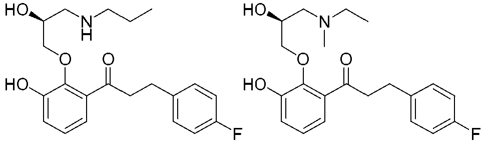 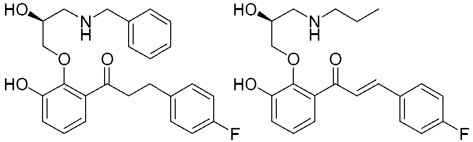 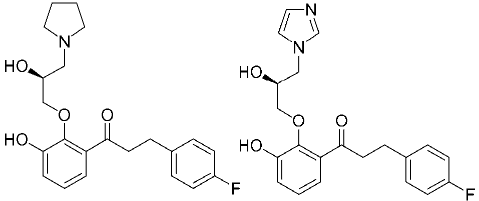 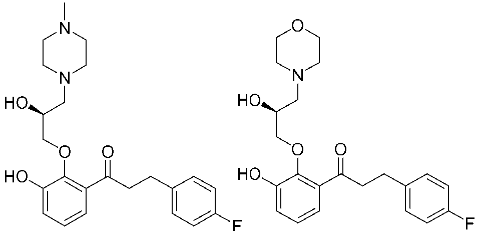 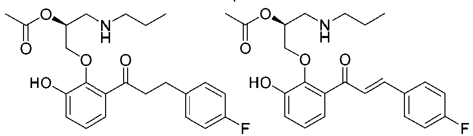 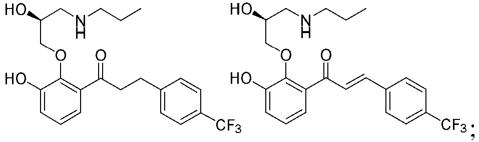
preferably, the isotope derivative has the following structures:

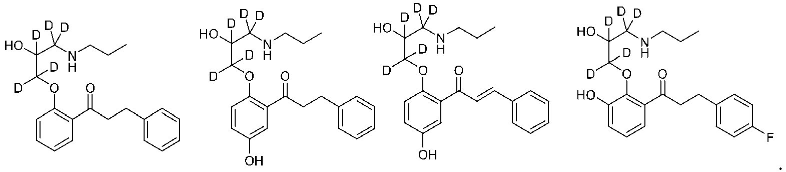

11. The use of any one of claims 1-10, wherein the product is a drug;
the drug is a topical preparation, an injection, or an oral preparation;
the topical preparation is selected from an aqueous solution, tincture, spirit, paste, oil, ointment, water-in-oil cream, oil ointment, oil-in-water cream, gel, film forming agent, paint, liniment, lotions, spray, suspension, solution, emulsion, microemulsion, suspension, plaster, gel, patch, powder aerosol, suppository, eye drops, or nasal drops;
the injection is selected from an aqueous, solution, suspension, emulsion, or powder injection;
the oral preparation is selected from a tablet, pill, dropping pill, powder, granule, capsule, troche, syrup, suspension, emulsion, microemulsion, powder aerosol, paste, sublingual tablet, or suppository;
preferably, the dosage form of the medicament is a topical preparation, in particular an ointment.

12. A topical preparation for treating and/or preventing psoriasis-like disease, wherein comprising the compound of any one of claims 1-11, or a pharmaceutically acceptable salt, stereoisomer, isotope derivative, solvate (such as hydrate), prodrug, metal chelate, crystal form, or metabolite thereof;
the topical preparation may also comprise a pharmaceutically acceptable excipient.

13. The topical preparation of claim 12, wherein the topical preparation is selected from an aqueous solution, tincture, spirit, paste, oil, ointment, water-in-oil cream, oil ointment, oil-in-water cream, gel, film forming agent, paint, liniment, lotions, spray, suspension, solution, emulsion, microemulsion, suspension, plaster, gel, patch, powder aerosol, suppository, eye drops, or nasal drops.

14. The topical preparation of claim 13, wherein the excipient comprises one or more of a solubilizer, oil phase, aqueous phase, and additive;
preferably, the solubilizer comprises one or more of fatty alcohol polyoxyethylene ether, fatty acid polyoxyethylene ester, castor oil polyoxyethylene ether, alkylphenol polyoxyethylene ether, fatty amine polyoxyethylene ether, polyoxyethylene polyoxypropylene copolymer, polyethylene glycol, polyethylene glycol stearate, sorbitol ester polyoxyethylene ether, sorbitan ester;
preferably, the oil phase comprises one or more of liquid paraffin, solid paraffin, stearic acid, white petrolatum, hexadecanol, octadecanol, glyceryl monostearate, isopropyl myristate, dimethicone, silicone, beeswax, lanolin, spermaceti, caprylic/capric triglyceride, isooctyl palmitate, isopropyl palmitate, PEG-100 stearate, vegetable oil, animal oil;
preferably, the aqueous phase comprises one or more of water, ethanol, glycerol, hexadecanol, octadecanol, hexylene glycol, propylene glycol, propanetriol, dipropylene glycol, butylene glycol, pentylene glycol;
preferably, the additive comprises one or more of an emulsifier, transdermal penetration enhancer, pH adjuster, preservative;
more preferably, the transdermal penetration enhancer comprises one or more of azone, dimethylsulfoxide, decylmethylsulfoxide, menthol, borneol, clove oil, ethanol, laurel oil, urea, capsaicin, oleic acid, urea;
more preferably, the pH adjusting agent comprises one or more of citric acid, salicylic acid, boric acid, fatty acid, sodium hydroxide, potassium hydroxide;
more preferably, the emulsifier is selected from one or more of triethanolamine, fatty acid soap, sodium lauryl sulfate, polyoxyethylene ether, polyoxypropylene ether, ethylene oxide, lecithin, gum arabic;
more preferably, the preservative comprises one or more of imidazolidinyl urea, diazoimidazolidinyl urea, DMDMH, quaternary ammonium salt-15, benzoic acid preservative and derivatives thereof, phenoxyethanol, benzyl alcohol, polyhydric alcohol preservatives, chlorphenesin, Bronopol, Kathon, benzalkonium bromide, benzalkonium chloride, ethylparaben, methylparaben, isopropanol, salicylic acid, sorbic acid;
and/or the additive further comprises one or more of vitamin E, dextran, betaine, butylated hydroxyanisole, ascorbic acid, iminodipropionic acid, ethanol, benzalkonium bromide, and hydroxypropanamide benzoic acid.

15. The topical preparation of claim 13, wherein when the topical preparation is a gel, the excipient further comprises one or more of gelatin, carbomer, methyl cellulose, sodium carboxymethyl cellulose, hydroxypropyl methyl cellulose, polyvinylpyrrolidone, polyvinyl alcohol, sodium alginate, glycerol, propylene glycol, dipropylene glycol, water.

16. The topical preparation of claim 13, wherein the topical preparation comprises 0.01-99.5% by weight of the compound of any one of claims 1-11, or a pharmaceutically acceptable salt, stereoisomer, isotopic derivative, solvate (e.g. hydrate), prodrug, metal chelate, crystalline form or metabolite thereof, preferably 0.02-0.5%, more preferably 0.1-0.5%.
